# EUROPÄISCHE PATENTSCHRIFT

(11) **EP 4 144 382 B1**
(45) Veröffentlichungstag und Bekanntmachung des Hinweises auf die Patenterteilung: **14.05.2025**
(21) Anmeldenummer: 22191023.5
(22) Anmeldetag: 18.08.2022
(51) Int. Cl.: A61L 2/14, A61G 11/00, A61L 2/24, A61M 16/00

(54) **VORRICHTUNG ZUR PLASMASTERILISATION MEDIZINTECHNISCHER GERÄTE**
DEVICE FOR PLASMA STERILIZATION OF MEDICAL DEVICES
DISPOSITIF DE STÉRILISATION PAR PLASMA D'APPAREILS MÉDICAUX

(30) Priorität: 01.09.2021 DE 102021004434; 14.09.2021 DE 102021004655
(43) Veröffentlichungstag der Anmeldung: 08.03.2023
(73) Patentinhaber: Löwenstein Medical Technology S.A., 2557 Luxembourg (LU)
(72) Erfinder: Pattai, Steffen, 56068 Koblenz (DE); Kurz, Torsten, 56337 Kadenbach (DE); Wellmann, Lukas, 56130 Bad Ems (DE)
(74) Vertreter: Löwenstein Medical IP

(56) Entgegenhaltungen:
- US-A1- 2011 027 125
- US-B1- 6 969 487

## Beschreibung

Die Erfindung betrifft eine Vorrichtung und ein Verfahren zur Sterilisation von medizintechnischen Geräten.

Beatmungsgeräte werden neben der Therapie von Schlafkrankheiten häufig auch im klinischen Bereich, insbesondere bei Atemwegserkrankungen, eingesetzt. Dabei ist es unvermeidbar, dass Teile der gasleitenden Komponenten, zum Beispiel die Patientengasleitungen, kontaminiert werden. Auch abseits der Kontamination durch Krankheitserreger ist eine hygienische Aufbereitung der Beatmungsgeräte nach Verwendung nötig.

Bekannte Verfahren zur hygienischen Aufbereitung sind dabei häufig aufwändig, zeitintensiv und erfordern oft auch eine zumindest teilweise Demontage der Geräte. Zudem sind die Reinigungsverfahren nicht selten kompliziert und erfordern aggressive Chemikalien um eine ausreichende Aufbereitung zu erreichen. Entsprechend stehen die Geräte nach einer Nutzung eine längere Zeit nicht zur Verfügung.

Die Aufgabe der vorliegenden Erfindung ist eine Vorrichtung zur Beatmung bereitzustellen, welche kostengünstig und mit geringem Aufwand aufbereitet werden kann. Die Aufgabe wird mit der Vorrichtung nach Anspruch 1 sowie dem Verfahren nach Anspruch 17 gelöst.

Die Erfindung betrifft ein System zur Sterilisation eines medizintechnischen Gerätes, umfassend zumindest ein medizintechnisches Gerät und zumindest eine Sterilisationsvorrichtung, wobei die Sterilisationsvorrichtung zumindest einen Plasmagenerator und zumindest eine Wasserquelle und/oder Wasserdampfquelle zur Bereitstellung von Wasser und/oder Wasserdampf umfasst. Die Sterilisationsvorrichtung ist dazu ausgebildet, plasmaaktivierten Wasserdampf zu generieren und, das System ist dazu ausgebildet, den plasmaaktivierten Wasserdampf zumindest teilweise durch das medizintechnische Gerät zu leiten

Erfindungsgemäss ist das System dadurch gekennzeichnet, dass das medizintechnische Gerät ein Beatmungsgerät ist, wobei das Beatmungsgerät zumindest eine Patientengasführung umfasst und das System dazu eingerichtet ist, den plasmaaktivierten Wasserdampf zumindest teilweise durch die Patientengasführung zu leiten.

In manchen Ausführungsformen ist das System dadurch gekennzeichnet, dass das System eine Abgasbehandlung umfasst, welche dazu eingerichtet und ausgebildet ist, den plasmaaktivierten Wasserdampf aufzubereiten.

In manchen Ausführungsformen ist das System dadurch gekennzeichnet, dass die Abgasbehandlung zumindest einen Filter und/oder einen Plasmagenerator umfasst.

In manchen Ausführungsformen ist das System dadurch gekennzeichnet, dass die Abgasbehandlung dazu eingerichtet und ausgebildet ist, aktive Spezies aus dem plasmaaktivierten Wasserdampf herauszufiltern.

In manchen Ausführungsformen ist das System dadurch gekennzeichnet, dass die Abgasbehandlung dazu eingerichtet und ausgebildet ist, langlebige aktive Spezies in kurzlebige Spezies umzuwandeln.

In manchen Ausführungsformen ist das System dadurch gekennzeichnet, dass die Sterilisationsvorrichtung in das Beatmungsgerät integriert ist.

In manchen Ausführungsformen ist das System dadurch gekennzeichnet, dass das Beatmungsgerät eine Atemgasquelle umfasst, wobei die Atemgasquelle mit der Sterilisationsvorrichtung verbunden ist und dazu eingerichtet ist, die Sterilisationsvorrichtung mit Arbeitsgas zu versorgen.

In manchen Ausführungsformen ist das System dadurch gekennzeichnet, dass die Sterilisationsvorrichtung zur Sterilisation mit einem Exspirationsanschluss und/oder einem Inspirationsanschluss der Patientengasführung verbindbar ist und das System so eingerichtet und ausgebildet ist, dass während der Sterilisation plasmaaktivierter Wasserdampf von der Sterilisationsvorrichtung durch den Exspirationsanschluss und/oder den Inspirationsanschluss in die Patientengasführung geleitet wird.

In manchen Ausführungsformen ist das System dadurch gekennzeichnet, dass die Sterilisationsvorrichtung während der Sterilisation so mit der Patientengasführung verbunden ist, dass der plasmaaktivierte Wasserdampf durch den Exspirationsanschluss in die Patientengasführung eingeleitet wird und durch den Inspirationsanschluss aus der Patientengasführung ausgeleitet wird, wobei die Patientengasführung so ausgebildet und eingerichtet ist, dass der plasmaaktivierte Wasserdampf bei der Strömung von Exspirationsanschluss zu Inspirationsanschluss die gesamte Patientengasführung durchströmt.

In manchen Ausführungsformen ist das System dadurch gekennzeichnet, dass das Beatmungsgerät eine Absaugeinheit umfasst, welche dazu eingerichtet und ausgebildet ist, den plasmaaktivierten Wasserdampf durch die Patientengasführung zu saugen.

In manchen Ausführungsformen ist das System dadurch gekennzeichnet, dass in das Beatmungsgerät eine Abgasbehandlung integriert ist, welche dazu eingerichtet und ausgebildet ist, den plasmaaktivierten Wasserdampf, welcher durch den Inspirationsanschluss und/oder den Exspirationsanschluss aus der Patientengasführung ausgeleitet wird, aufzubereiten.

In manchen Ausführungsformen ist das System dadurch gekennzeichnet, dass die Abgasbehandlung in das Beatmungsgerät integriert ist, wobei die Abgasbehandlung dazu eingerichtet und ausgebildet ist, den plasmaaktivierten Wasserdampf, welcher durch den Inspirationsanschluss und/oder den Exspirationsanschluss aus der Patientengasführung ausgeleitet wird, aufzubereiten.

In manchen Ausführungsformen ist das System dadurch gekennzeichnet, dass die Sterilisationsvorrichtung und die Abgasbehandlung innerhalb des Beatmungsgerätes mit der Patientengasführung so verbunden ist, dass bei der Sterilisation plasmaaktivierter Wasserdampf von durch die gesamte Patientengasführung strömt.

In manchen Ausführungsformen ist das System dadurch gekennzeichnet, dass die Sterilisationsvorrichtung dazu ausgebildet ist, den plasmaaktivierten Wasserdampf durch die Patientengasführung zu fördern.

In manchen Ausführungsformen ist das System dadurch gekennzeichnet, dass in dem System eine Benutzerschnittstelle angeordnet ist und über die Benutzerschnittstelle eine Dauer und/oder ein Modus der Sterilisation einstellbar ist.

In manchen Ausführungsformen ist das System dadurch gekennzeichnet, dass die Benutzerschnittstelle in das Beatmungsgerät integriert ist.

In manchen Ausführungsformen ist das System dadurch gekennzeichnet, dass das System dazu eingerichtet und ausgebildet ist, die Sterilisation nach Starten über die Benutzerschnittstelle automatisch durchzuführen.

In manchen Ausführungsformen ist das System dadurch gekennzeichnet, dass das Beatmungsgerät dazu eingerichtet ist, die Voraussetzungen zum Start der Sterilisation zu prüfen und nur dann die Sterilisation freigibt, wenn alle Voraussetzungen erfüllt sind.

In manchen Ausführungsformen ist das System dadurch gekennzeichnet, dass die Benutzerschnittstelle dazu eingerichtet ist einen Status der Sterilisation anzuzeigen.

In manchen Ausführungsformen ist das System dadurch gekennzeichnet, dass die Sterilisationsvorrichtung dazu eingerichtet ist in einem ersten Sterilisationsmodus einen dauerhaften Strom an plasmaaktiviertem Wasserdampf bereitzustellen und/oder durch die Patientengasführung zu fördern.

In manchen Ausführungsformen ist das System dadurch gekennzeichnet, dass die Sterilisationsvorrichtung dazu eingerichtet ist, in einem zweiten Sterilisationsmodus einen gepulsten Strom an plasmaaktiviertem Wasserdampf bereitzustellen und/oder durch die Patientengasführung zu fördern.

In manchen Ausführungsformen ist das System dadurch gekennzeichnet, dass der erste und/oder zweite Sterilisationsmodus über die Benutzerschnittstelle wählbar ist.

In manchen Ausführungsformen ist das System dadurch gekennzeichnet, dass das medizintechnische Gerät ein Inkubator ist, wobei der Inkubator zumindest einen Inkubationsraum aufweist und der Inkubationsraum zumindest eine Zuleitung und eine Ableitung verfügt und über die Zuleitung plasmaaktivierter Wasserdampf in den Inkubationsraum geleitet wird und über die Ableitung abgleitet wird.

In manchen Ausführungsformen ist das System dadurch gekennzeichnet, dass im Inkubator zumindest eine Umwälzeinheit angeordnet ist, welche dazu ausgebildet ist den plasmaaktivierten Wasserdampf im Inkubationsraum umzuwälzen.

Die Erfindung betrifft auch ein Verfahren zur Sterilisation eines medizintechnischen Gerätes. Das Verfahren ist dadurch gekennzeichnet, dass über eine Sterilisationsvorrichtung ein plasmaaktivierter Wasserdampf generiert wird und der plasmaaktivierte Wasserdampf zumindest teilweise durch das medizintechnische Gerät geleitet wird.

In manchen Ausführungsformen kennzeichnet das Verfahren, dass das medizintechnische Gerät ein Beatmungsgerät ist und der plasmaaktivierte Wasserdampf zumindest teilweise durch eine Patientengasführung des Beatmungsgerätes geleitet wird.

In manchen Ausführungsformen kennzeichnet das Verfahren, dass der plasmaaktivierte Wasserdampf durch die gesamte Patientengasführung geleitet wird, wobei der plasmaaktivierte Wasserdampf nach Strömung durch die Patientengasführung durch eine Abgasbehandlung geleitet wird.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Ein medizintechnisches Gerät kann jedwedes Gerät für eine medizintechnische Anwendung angesehen werden. Insbesondere sind im Zuge der Erfindung Beatmungsgeräte und Inkubatoren gemeint.

Zu den Inkubatoren können neben Inkubatoren, sogenannten Brutkästen, auch Wärmebetten für Neugeborene zählen.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Im Zuge der Erfindung kann Atemgas jedwede Art von Gas und/oder Gasmischung sein, welche durch einen Patienten eingeatmet werden kann, ohne diesem zu Schaden. Beispielsweise kann als Atemgas Umgebungsluft genutzt werden. Auch synthetische Luft und/oder Luft aus einer Druckgasleitung bzw. Druckgasflaschen kann als Atemgas angesehen werden. Auch Mischungen aus Stickstoff und Sauerstoff und optional weiteren Gasen ist als Atemgas verwendbar. In manchen Ausführungsformen kann dem Atemgas auch ein oder mehrere Anästhesiegase zugesetzt werden. Als Atemgasquelle wird im Zuge der Erfindung ganz allgemein eine Gasquelle bezeichnet, über welche Atemgas gefördert wird. Beispielsweise kann eine Atemgasquelle auch einen Gasmischer umfassen, um einem Atemgas zum Beispiel Sauerstoff und/oder Anästhesiegas beizumischen. Das Atemgas kann beispielsweise durch eine Fördereinheit zu einem Patienten gefördert werden. In manchen Ausführungsformen ist die Fördereinheit gleichzusetzen mit der Atemgasquelle, beispielsweise als Gebläse, welches Umgebungsluft in das Beatmungsgerät fördert. Atemgasquelle und Fördereinheit können durchaus auch getrennt sein, beispielsweise bei Beatmungsgeräten mit einem (teilweise) geschlossenen Gaskreislauf. Die Fördereinheit ist dabei so ausgelegt, dass das Patientengas innerhalb des Kreislaufs gefördert wird. Als Patientengas ist dabei das Gas bzw. die Gasmischung zu verstehen, welches innerhalb des Kreislaufs gefördert wird. Das Gas bzw. die Gasmischung, welche dem Gaskreislauf zugeführt wird, beispielsweise um verbrauchten Sauerstoff und/oder andere Gase zu ersetzen, wird im Zuge der Erfindung als Frischgas bezeichnet.

Das erfinderische System ist dazu ausgelegt, dass ein in dem System enthaltenes Beatmungsgerät effektiv und kosten- und zeitgünstig hygienisch aufbereitet werden kann. Die Sterilisationsvorrichtung ist dazu beispielhaft dazu eingerichtet einen plasmaaktivierten Wasserdampf zu erzeugen. Dazu wird in einen Plasmastrom eine geringe Menge an Wasser und/oder Wasserdampf injiziert. Gegenüber einem reinen Plasma sind im plasmaaktivierten Wasserdampf auch langlebige aktive Spezies enthalten, welche zum Beispiel eine längere Wirkungszeit in dem Beatmungsgerät ermöglichen. Langlebige aktive Spezies sind beispielsweise Ozon und/oder Hydroxyl. Langlebige Spezies zerfallen im Mittel nicht unter 60 Sekunden. Erfindungsgemäß ist daran gedacht, dass zumindest die Patientengasführung, insbesondere der Teil, welcher mit der ausgeatmeten Luft des Patienten ungefiltert in Kontakt kommt, durch den plasmaaktivierten Wasserdampf hygienisch aufbereitet wird.

Um zu vermeiden, dass die aktiven Spezies in die Umgebungsluft gelangen, ist in manchen Ausführungsformen vorgesehen, dass der plasmaaktivierte Wasserdampf nach der Durchströmung der Patientengasführung durch eine Abgasbehandlung geleitet wird, etwa einen Filter und/oder einen zweiten Plasmagenerator, welcher die langlebigen Spezies in kurzlebige Spezies umwandelt. Kurzlebige Spezies zerfallen im Mittel innerhalb von <60 Sekunden. Ist in dem Beatmungsgerät eine Abluft vorgesehen, durch welche die ausgeatmete Luft des Patienten während der Beatmung aus dem Beatmungsgerät geleitet wird, kann vorgesehen sein, dass die Abgasbehandlung vor und/oder in den Abluftweg integriert wird.

Zur Förderung des plasmaaktivierten Wasserdampfs durch die Patientengasleitung kann beispielsweise eine Absaugeinheit genutzt werden, welche in das Beatmungsgerät integriert sein kann. Durch diese Absaugeinheit kann nach der Sterilisation auch weiter Gas durch die Patientengasführung gesaugt werden und/oder ein Unterdruck erzeugt werden, um die Patientengasführung möglichst frei von plasmaaktiviertem Wasserdampf zu bekommen. Es ist zudem angedacht, dass zu diesem Zweck auch ein Nachspülen mit Atemgas, Frischgas oder Arbeitsgas durch die Patientengasführung angewendet werden kann.

Um gegenüber Plasma und/oder plasmaaktivierten Wasserdampf empfindliche Komponenten nicht zu beschädigen ist vorgesehen, dass diese während der Sterilisation beispielsweise über Ventile von der Patientengasführung abgekoppelt werden.

Das Arbeitsgas für die Plasmaerzeugung wird beispielsweise über eine externe Gasquelle bereitgestellt, kann aber alternativ oder ergänzend auch durch die Atemgasquelle des Beatmungsgerätes bereitgestellt werden.

In manchen Ausführungsformen des Systems ist das Beatmungsgerät als Anästhesiegerät ausgeführt und umfasst neben der Patientengasführung für eine maschinelle Beatmung eine, gegebenenfalls mit der maschinellen Patientengasführung verbundene, Patientengasführung für eine manuelle Beatmung (z.B. über einen Beatmungsbeutel). Während der maschinellen Beatmung ist eine Förderung von Patientengas in der Regel nur durch eine von den beiden Patientengasführungen möglich, während die jeweils andere beispielsweise durch Ventile verschlossen wird. Für die Sterilisation kann vorgesehen sein, dass die Ventile so geöffnet werden, dass eine gleichzeitige Durchströmung beider Patientengasführungen (für die maschinelle sowie die manuelle Beatmung) möglich ist. Alternativ oder ergänzend kann auch ein Sterilisationsmodus vorgesehen sein, bei dem die Patientengasführungen nach einander oder alternierend von plasmaaktiviertem Wasserdampf durchströmt werden.

Es kann durchaus auch vorgesehen sein, dass das Beatmungsgerät ein sehr rudimentäres Gerät ist, welches im Wesentlichen eine Patientengasführung und eine Atemgasquelle umfasst. Umfasst das System eine solche Ausführung des Beatmungsgerätes, kann vorgesehen sein, dass die Sterilisationsvorrichtung vollständig unabhängig vom Beatmungsgerät operiert, also über eine eigene Energieversorgung und Gasversorgung verfügt und auch unabhängig vom Beatmungsgerät gesteuert wird. Beispielsweise wird dann das Beatmungsgerät so mit der Sterilisationsvorrichtung verbunden, dass durch die Sterilisationsvorrichtung der plasmaaktivierte Wasserdampf durch die Patientengasführung des Beatmungsgerätes geführt wird.

Gesteuert wird die Sterilisation beispielsweise über die Steuereinheit des Beatmungsgerätes. Soweit die Sterilisationsvorrichtung 20 nicht in das Beatmungsgerät integriert ist, ist dazu eine Verbindung über Schnittstellen zwischen dem Beatmungsgerät und der Sterilisationsvorrichtung vorgesehen. Es ist vorgesehen, dass über die Benutzerschnittstelle Eingaben zur Sterilisation möglich sind, beispielsweise die Dauer und/oder einen Modus betreffend. In manchen Ausführungsformen kann auch vorgesehen sein, dass der Fluss und/oder eine mittlere Verweilzeit des plasmaaktivierten Wasserdampfs in der Patientengasführung einstellbar ist. Auch die Menge an in den Plasmastrom injiziertem Wasser bzw. Wasserdampf kann einstellbar sein. Weitere Einstellmöglichkeiten zur Sterilisation können beispielsweise auch ein Nachspülen der Patientengasführung mit Arbeitsgas und/oder Frischgas und/oder Atemgas betreffen.

Über die Steuereinheit werden beispielhaft die Ventile des Beatmungsgerätes so geschaltet, dass der plasmaaktivierte Wasserdampf ausschließlich durch die Patientengasführung gefördert wird. Auch wird über die Steuereinheit die Zufuhr von Arbeitsgas zur Sterilisationsvorrichtung gesteuert. In manchen Ausführungsformen sind die Sensoreinheit und/oder die Erkennungseinheit so ausgebildet und eingerichtet, dass die Sterilisation überwacht wird. Beispielsweise kann vorgesehen sein, dass bei einer erkannten Leckage, durch welche plasmaaktivierter Wasserdampf aus dem System und/oder dem Beatmungsgerät austreten kann die Sterilisation unterbrochen wird und gegebenenfalls ein Alarm generiert wird.

Neben einer Steuerung über das Beatmungsgerät kann die Sterilisationsvorrichtung auch über eigene Steuereinheiten verfügen, welche beispielsweise die Erzeugung des plasmaaktivierten Wasserdampfs und/oder dessen Förderung/Leitung durch die Patientengasführung zumindest teilweise steuern. Auch kann vorgesehen sein, dass an der Sterilisationsvorrichtung eine Benutzerschnittstelle angeordnet ist. Über diese Benutzerschnittstelle können beispielsweise Eingaben zur Steuerung der Sterilisationsvorrichtung gemacht werden. Zumindest ist vorgesehen, dass über diese optionale Benutzerschnittstelle die Sterilisationsvorrichtung ein- und ausgeschaltet werden kann.

Ist keine Absaugeinheit in dem Beatmungsgerät integriert, ist vorgesehen, dass der plasmaaktivierte Wasserdampf durch den Druck des Arbeitsgases der Sterilisationsvorrichtung durch die Patientengasführung gefördert wird. Alternativ oder ergänzend ist angedacht, dass die Förderung des plasmaaktivierten Wasserdampfs durch die Fördereinheit und/oder eine zusätzliche Fördereinheit nur für die Sterilisation, etwa ein Gebläse, zumindest unterstütz wird.

Die Erfindung betrifft ferner auch ein Verfahren zur hygienischen Aufbereitung eines Beatmungsgerätes.

Das Verfahren umfasst dabei zumindest die Schritte der Erzeugung eines plasmaaktivierten Wasserdampfs und die Förderung des plasmaaktivierten Wasserdampfs durch zumindest Teile der Patientengasführung des Beatmungsgerätes.

Die Erzeugung des plasmaaktivierten Wasserdampfs lässt sich unterteilen in einen ersten Schritt, in dem in einem Plasmagenerator aus einem Arbeitsgas ein kalter Plasmastrom erzeugt wird und in einen zweiten Schritt, in welchem geringe Mengen an Wasser in den Plasmastrom injiziert werden, wodurch der plasmaaktivierte Wasserdampf entsteht.

Zur Förderung des plasmaaktivierten Wasserdampfs durch die Patientengasführung, wird der plasmaaktivierte Wasserdampf in die Patientengasführung geleitet. In manchen Ausführungsformen ist die Patientengasführung so ausgebildet und eingerichtet, dass der plasmaaktivierte Wasserdampf über einen Anschluss eingeleitet wird und über einen anderen Anschluss aus der Patientengasführung ausgeleitet wird, wobei der plasmaaktivierte Wasserdampf dabei die komplette Patientengasführung durchströmt.

In einem optionalen weiteren Verfahrensschritt wird der plasmaaktivierte Wasserdampf, nachdem er durch die Patientengasführung geströmt ist, über eine Abgasbehandlung aufbereitet. Die Aufbereitung kann dabei ein Herausfiltern von langlebigen aktiven Spezies sein und/oder eine Plasmabehandlung, um die langlebigen aktiven Spezies in kurzlebige Spezies umzuwandeln.

Optional kann sich an die Sterilisation ein Spülen der Patientengasführung mit Arbeitsgas, Frischgas und/oder Atemgas anschließen.

In manchen Ausführungsformen wird durch das Beatmungsgerät überprüft, ob alle Voraussetzungen erfüllt sind, dass eine Sterilisation gestartet werden kann. Unter Voraussetzungen, die erfüllt werden müssen, sind beispielsweise eine erfolgte Verbindung der Sterilisationsvorrichtung mit der Patientengasführung, eine Versorgung der Sterilisationsvorrichtung mit Arbeitsgas und Wasser, eine Verbindung der Patientengasführung mit der Absaugeinheit und/oder der Abgasbehandlung, keine Verbindung zwischen Patientengasführung und einem Patienten, Stromversorgung von Beatmungsgerät und Sterilisationsvorrichtung. Auch eine Dichtigkeitsprüfung des Systems bzw. der Atemgasführung kann vorgesehen sein.

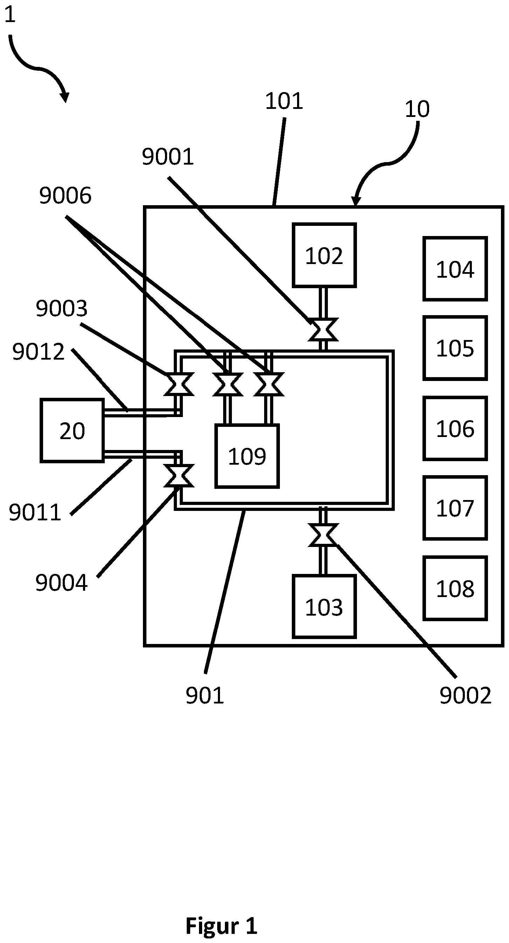

In den Figuren 1 bis 5 wird die Erfindung anhand beispielhafter Ausführungsformen näher erläutert. Es ist darauf hinzuweisen, dass die gezeigten Ausführungsformen in einem beispielhaften Zustand dargestellt sind, welcher für die Sterilisation hergestellt wird, in der Regel nicht aber während der Beatmung eines Patienten. Zu manchen Ausführungsformen wird zusätzlich der Aufbau beschrieben, welcher für eine Beatmung eines Patienten hergestellt wird.

Figur 1 zeigt eine beispielhafte Ausführungsform des Systems, wobei das Beatmungsgerät 10 als Anästhesiegerät ausgebildet ist und über einen zumindest teilweise geschlossenen Patientengaskreislauf verfügt. Das Beatmungsgerät 10 verfügt beispielhaft zumindest über ein Gehäuse 101, in welchem eine Atemgasquelle 102, eine Fördereinheit 103, eine Steuereinheit 104, eine Sensoreinheit 105, eine Auswerteeinheit 106, eine Benutzerschnittstelle 107, eine Erkennungseinheit 108 sowie ein CO2-Absorber 109 angeordnet sind. In dem Gehäuse 101 des Beatmungsgerätes 10 ist eine Patientengasführung 901 angeordnet, durch welche das Patientengas geleitet wird. Über einen Inspirationsanschluss 9011 kann das Patientengas über ein Patienteninterface, beispielsweise eine Beatmungsmaske, zum Patienten geleitet werden.

Das vom Patienten ausgeatmete Gas wird über den Exspirationsanschluss 9012 vom Patienten kommend wieder in die Patientengasführung 901 im Beatmungsgerät 10 geleitet. An die Patientengasführung 901 sind die Atemgasquelle 102, die Fördereinheit 103 und der CO2-Absorber 109 angeschlossen, wobei zwischen der Patientengasführung 901 und den angeschlossenen Komponenten beispielhaft die Ventile 9001, 9002, und 9006 angeordnet sind. Je nach Bedarf können die Ventile 9001, 9002 und 9006 geöffnet, geschlossen oder partiell geöffnet bzw. geschlossen werden.

Neben den aufgezählten Komponenten können selbstverständlich auch weitere Bestandteile in dem Beatmungsgerät 10 enthalten sein, beispielsweise eine Gasflusskompensation (etwa ein Beatmungsbeutel) oder Anschlüsse und/oder Patientengaswege zur manuellen Beatmung.

Die Atemgasquelle 102 ist beispielhaft so eingerichtet, dass hierüber bei Bedarf Frischgas bzw. Atemgas in den Patientengaskreislauf bzw. die Patientengasführung 901 eingespeist werden kann. Dazu ist die Atemgasquelle 102 beispielsweise mit einer Druckluftquelle verbunden, beispielsweise einer Druckluftflasche und/oder einem Hausanschluss (Krankenhaus). Zudem ist eine Sauerstoffquelle, ebenfalls über eine Druckgasflasche oder einem Hausanschluss, vorgesehen sowie optional auch zumindest eine Quelle für ein Anästhesiegas. Die Zufuhr von Frischgas in die Patientengasführung wird beispielsweise über das Ventil 9001 sowie über die Atemgasquelle 102 gesteuert.

Die Fördereinheit 103 ist dazu eingerichtet, einen Atemgas- bzw. Patientengasstrom durch die Patientengasführung 901 zu fördern. Beispielsweise ist die Fördereinheit 103 als ein Balg eingerichtet, welcher durch Kontraktion, z.B. durch ein Antriebsgas angetrieben, das Patientengas durch die Patientengasführung 901 zum Patienten fördert. Durch die Exspiration des Patienten wird der Balg wieder expandiert und der Atemzyklus kann erneut beginnen. In manchen Ausführungsformen ist die Fördereinheit 103 zusätzlich oder alternativ als Gebläse ausgeführt.

Die Steuereinheit 104 ist dazu eingerichtet zumindest die Atemgasquelle 102, die Fördereinheit 103 sowie die Ventile 9001, 9002, 9003, 9004, 9006 zu steuern. Die Steuereinheit 104 kann beispielsweise dazu in mehrere Steuereinheiten unterteilt sein, welche jeweils zur Steuerung einer Komponente des Beatmungsgerätes 10 und/oder des Systems 1 eingerichtet sind. In manchen Ausführungsformen ist die Steuereinheit 104 dazu eingerichtet auch die Sterilisationsvorrichtung 20 zu steuern. Die Steuereinheit 104 ist beispielhaft auch dazu eingerichtet, Werte, Daten und Informationen von der Sensoreinheit 105, der Auswerteeinheit 106, der Benutzerschnittstelle 107 und/oder der Erkennungseinheit 108 zu verarbeiten und/oder als Grundlage der Steuerung der Komponenten des Beatmungsgerätes 10 und/oder des Systems 1 zu nutzen.

Die Sensoreinheit 105 ist beispielsweise dazu eingerichtet um über mit ihr verbundene Sensoren Messwerte und Messdaten zu erfassen, welche beispielsweise in dem Beatmungsgerät 10 oder allgemein in dem System 1 angeordnet sind. Diese Messwerte und Messdaten können beispielsweise die Beatmung des Patienten, wie zum Beispiel Fluss, Druck, Gaszusammensetzung, Temperatur, Konzentration des Blutsauerstoffs, Herzschlag und/oder Feuchtigkeit umfassen. Auch weitere, optional auch nicht direkt mit der Beatmung zusammenhängende Daten und Werte, können beispielhaft über die Sensoreinheit 105 erfasst werden.

Die Auswerteeinheit 106 ist beispielhaft dazu ausgebildet, unter anderem die Messwerte und/oder Messdaten der Sensoreinheit 105 und/oder anderer Datenquellen, wie zum Beispiel über eine Benutzerschnittstelle 107 eingegebene Daten und Informationen, aufzubereiten, weiterzuverarbeiten, zu analysieren und/oder zu bewerten.

Im System 1 bzw. beispielhaft im Beatmungsgerät 10 ist eine Benutzerschnittstelle 107 angeordnet, welche zumindest eine Eingabevorrichtung und/oder Anzeigevorrichtung umfasst. Über die Eingabevorrichtung, beispielsweise eine Tastatur und/oder (Dreh-)Knöpfe und/oder eine berührungsempfindliche Oberfläche (Touchscreen) können Daten, Informationen und Werte eingegeben werden und auch Vorgaben zur Steuerung des Beatmungsgerätes 10 und/oder des Systems 1. Eine Anzeigevorrichtung kann beispielsweise ein Display sein oder auch eine analoge Anzeige oder auch einfache Leuchtsymbole. Die Anzeigeeinrichtung ist beispielsweise dazu eingerichtet, Daten und/oder Werte und/oder Informationen anzuzeigen, welche unter anderem den Status des Systems 1 und/oder die Beatmung eines Patienten betreffen. In manchen Ausführungsformen sind über die Benutzerschnittstelle 107 zudem Eingaben zur Steuerung der Sterilisationsvorrichtung 20 möglich.

Die Erkennungseinheit 108 ist in beispielhaft dazu eingerichtet, technische Probleme des Beatmungsgerätes 10 zu erfassen. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Auswerteeinheit 108 kann bei einem erkannten technischen Problem einen Alarm auf dem Beatmungsgerät 10 und/oder über eine Schnittstelle (nicht dargestellt) anzeigen und/oder übermitteln. In manchen Ausführungsformen ist die Erkennungseinheit 107 auch dazu eingerichtet technische Probleme des gesamten Systems 1 zu erfassen, beispielsweise einer extern des Beatmungsgerätes 10 angeordneten Sterilisationsvorrichtung 20, welche beispielsweise mit dem Beatmungsgerät 10 verbunden ist.

Der CO2-Absorber 109 des Beatmungsgerätes 10 ist dazu eingerichtet zumindest teilweise CO2 aus dem Patientengas herauszufiltern. Beispielsweise ist der CO2-Absorber 109 mit einem Material gefüllt, welches CO2 absorbieren kann. Dazu wird zum Beispiel das Patientengas so geleitet, dass es das Material in dem CO2-Absorber 109 umströmt bzw. durchströmt.

Bei dem teilweise geschlossenen Patientengaskreislauf wird das Patientengas durch die Fördereinheit 103 durch die Patientengasführung 901 über den Inspirationsanschluss 9011 zum Patienten gefördert. Das vom Patienten ausgeatmete Patientengas wird durch den Exspirationsanschluss 9012 wieder in die Patientengasführung 901 des Beatmungsgerätes 10 geleitet. Die Flussrichtung des Patientengases innerhalb der Patientengasführung 901 wird beispielsweise über die Ventile 9003 und 9004 geregelt, sodass beispielsweise während der Inspiration das Ventil 9003 geschlossen ist, sodass das Patientengas nur über den Inspirationsanschluss 9011 zum Patienten gefördert werden kann. Dahingegen wird das Ventil 9004 während der Exspiration des Patienten geschlossen, sodass die ausgeatmete Luft durch den Exspirationsanschluss 9012 in die Patientengasführung 901 geleitet wird. In manchen Ausführungsformen sind insbesondere die Ventile 9003 und 9004 als Rückschlagventile ausgeführt. Beispielhaft sind zwischen der Patientengasführung 901 und der Atemgasquelle 102, der Fördereinheit 103 und dem CO2-Absorber 109 die Ventile 9001, 9002 und 9006 angeordnet. Das Patientengas, welches von dem Patienten ausgeatmet wird, wird beispielhaft durch einen CO2-Absorber 109 geleitet, um das ausgeatmete CO2 aus dem Patientengas zu entfernen. Beispielsweise um den verbrauchten Sauerstoff und/oder auch verbrauchtes Anästhesiegas nachzuliefern kann über die Atemgasquelle 102 Frischgas bzw. eine entsprechende Gasmischung in den Gaskreislauf eingebracht werden.

In der in Figur 1 beispielhaft dargestellten Ausführungsform des Systems 1 ist eine Sterilisationsvorrichtung 20 mit dem Beatmungsgerät 10 verbunden. Beispielhaft wird die Sterilisationsvorrichtung 20 über den Inspirationsanschluss 9011 und den Exspirationsanschluss 9012 mit der Patientengasführung 901 des Beatmungsgerätes 10 verbunden.

Zur Sterilisation beziehungsweise hygienischen Aufbereitung des Beatmungsgerätes 10 ist in dem System 1 eine Sterilisationsvorrichtung 20 angeordnet. In der dargestellten beispielhaften Ausführungsform ist die Sterilisationsvorrichtung 20 außerhalb des Gehäuses 101 des Beatmungsgerätes 10 angeordnet und gasleitend über den Inspirationsanschluss 9011 und den Exspirationsanschluss 9012 mit der Patientengasführung 901 verbunden.

Die Sterilisationsvorrichtung 20 umfasst zumindest einen Plasmagenerator. Aus einem Arbeitsgas wird über den Plasmagenerator ein kaltes Plasma erzeugt. Beispielsweise wird hier eine Standard-Verfahren zur Erzeugung eines kalten Plasmas verwendet. Der Plasmagenerator ist dabei so ausgebildet, dass eine Bogenentladung verhindert wird. Beispielsweise ist der Plasmagenerator dazu eingerichtet über eine stille elektrische Entladung, auch bekannt als dielektrische Barriereentladung oder "Dielectric Barrier Discharge" (DBD), ein kaltes Plasma zu erzeugen. Dazu wird an zwei Elektroden eine Hochspannung, beispielsweise eine sinusförmige Wechselspannung oder eine gepulste Gleichspannung, angelegt. Zwischen den Elektroden befindet sich ein Gasraum, wobei eine der Elektroden durch eine dielektrische Schicht von dem Gasraum getrennt ist. In einer Folge der angelegten Hochspannung kommt es zur Ionisation zumindest eines Teiles des Gases in dem Gasraum zwischen den Elektroden und darauffolgend zu einer sogenannten Gasentladung und Entstehung des Plasmas.

Als Arbeitsgas der Sterilisationsvorrichtung 20 kann beispielsweise Umgebungsluft dienen, welche mit einem Druck in einem Bereich zwischen 2 bar und 10 bar, insbesondere zwischen 4 bar und 6 bar bereitgestellt wird. In manchen Ausführungsformen kann auch synthetische Luft und/oder Druckluft und/oder andere Gasgemische als Arbeitsgas genutzt werden. Das Arbeitsgas wird beispielsweise durch eine, extra mit der Sterilisationsvorrichtung 20 verbundenen, Gasversorgung, etwa Druckgasflaschen und/oder einem Kompressor und/oder einer Hausgasleitung, zur Verfügung gestellt. In manchen Ausführungsformen des Systems 1 wird das Arbeitsgas vom Beatmungsgerät 10 geliefert, zum Beispiel zumindest teilweise aus der Atemgasquelle 102, welche über eine extra Leitung mit der Sterilisationsvorrichtung 20 verbunden wird. Die Gasmischung kann dabei beispielsweise angepasst werden, insbesondere wenn die Atemgasquelle 102 zur Arbeitsgasversorgung genutzt wird, kann das Gasgemisch so eingestellt werden, dass keine Anästhesiegase dem Arbeitsgas beigemischt werden.

In der beispielhaften Ausführungsform wird in den entstehenden Plasmastrom eine geringe Menge Wasser, beispielsweise <5 ml pro Minute, injiziert, um einen plasmaaktivierten Wasserdampf zu erzeugen. Dazu umfasst die Sterilisationsvorrichtung 20 zumindest eine Wasserquelle. Die Wasserquelle kann zum Beispiel von einem Wasseranschluss oder einem integrierten Wassertank gespeist werden. In manchen Ausführungsformen wird das Wasser in einem feinen Strahl in das Plasma injiziert. Auch eine tropfenweise Injektion oder Verneblung von Wasser ist denkbar. In manchen Ausführungsformen wird das Wasser als Wasserdampf in den Plasmastrom injiziert. Dazu umfasst die Sterilisationsvorrichtung 20 optional einen Verdampfer. Auch die Verwendung von Wasserdampf zumindest als Teil des Arbeitsgases ist grundsätzlich denkbar. Dazu wird dem Arbeitsgas, welches in den Plasmagenerator geleitet wird, bereits Wasserdampf zugesetzt bzw. besteht ausschließlich aus Wasserdampf.

Beispielsweise über den Exspirationsanschluss 9012 wird der plasmaaktivierte Wasserdampf in die Patientengasführung 901 geleitet. Die Förderung des plasmaaktivierten Wasserdampfs erfolgt beispielsweise primär durch den angelegten Druck des Arbeitsgases. In der beispielhaft dargestellten Ausführungsform in Figur 1 wird der plasmaaktivierte Wasserdampf durch den Inspirationsanschluss 9011 aus der Patientengasführung 901 ausgeleitet. Während der Sterilisation sind die Ventile 9001, 9002 und 9006 geschlossen, damit der plasmaaktivierte Wasserdampf nicht in Kontakt mit Komponenten des Beatmungsgerätes 10 kommt, welche zum Beispiel keine ausreichende Korrosionsbeständigkeit aufweisen oder anderweitig Schaden durch den plasmaaktivierten Wasserdampf nehmen können. In manchen Ausführungsformen sind zumindest die Fördereinheit 103 und/oder der CO2-Absorber 109 so eingerichtet und ausgebildet, dass diese vom Beatmungsgerät 10 getrennt und ausgetauscht werden können. In manchen Ausführungsformen sind die Fördereinheit 103 und/oder der CO2-Absorber so eingerichtet und ausgebildet, dass auch diese mit dem plasmaaktivierten Wasserdampf hygienisch aufbereitet werden können.

Das Einleiten des plasmaaktivierten Wasserdampfs durch den Exspirationsanschluss 9012 und das Ausleiten durch den Inspirationsanschluss 9011 sorgt dafür, dass der gesamte Kreislauf, insbesondere die Patientengasführung 901, mit dem plasmaaktivierten Wasserdampf hygienisch aufbereitet werden kann. Die Strömungsrichtung ist dabei beispielhaft so eingerichtet und ausgebildet, wie auch die Strömungsrichtung des Patientengases während der Beatmung, welches durch der Exspirationsanschluss 9012 vom Patienten in die Patientengasführung 901 eingeleitet wird und in der Patientengasführung 901 bis zum Inspirationsanschluss 9011 geleitet wird. In manchen Ausführungsformen wird der plasmaaktivierte Wasserdampf entgegengesetzt geleitet, also vom Inspirationsanschluss 9011 durch die Patientengasführung 901 zum Exspirationsanschluss 9012.

Die Ventile 9003 und 9004 sind während der Sterilisation beispielhaft geöffnet, sodass der plasmaaktivierte Wasserdampf frei durch die Patientengasführung 901 strömen kann. Beispielhaft ist vorgesehen, dass der plasmaaktivierte Wasserdampf über den Exspirationsanschluss 9012 in die Patientengasführung 901 eingeleitet und über den Inspirationsanschluss 9011 aus der Patientengasführung 901 ausgeleitet wird. In manchen Ausführungsformen kann es auch vorgesehen sein, dass die entgegensetze Strömungsrichtung gewählt wird oder die Strömungsrichtung periodisch nach einiger Zeit gewechselt wird.

Wird der Druck des Arbeitsgases als Haupttriebkraft genutzt um den plasmaaktivierten Wasserdampf in und/oder durch die Patientengasführung zu leiten/fördern, kann es beispielsweise vorgesehen sein, dass zunächst das Plasma und/oder der plasmaaktivierte Wasserdampf erzeugt wird, während das Ventil 9003 hinter dem Exspirationsanschluss 9012 geschlossen bleibt. Wird das Ventil 9003 dann geöffnet kann der plasmaaktivierte Wasserdampf beispielsweise durch einen Druckgradienten zwischen Sterilisationsvorrichtung 20 und Patientengasführung 901 in die Patientengasführung 901 strömen. Es kann alternativ oder ergänzend auch vorgesehen sein, dass der Druck des Arbeitsgases dauerhaft aufrechterhalten wird und durch den Druck der plasmaaktivierte Wasserdampf beständig durch die Patientengasführung 901 strömt.

Da der plasmaaktivierte Wasserdampf einige langlebige Spezies wie Ozon und Hydroxyl aufweisen kann, kann eine Nachreinigung des plasmaaktivierten Wasserdampfs nach Durchströmen der Patientengasführung 901 nötig sein. Ziel ist es dabei, dass diese langlebigen Spezies nicht in die Umgebungsluft abgegeben werden und beispielsweise das, mit der Reinigung beschäftigte, Personal schädigt. Dazu der plasmaaktivierte Wasserdampf durch den Inspirationsanschluss 9011 aus der Patientengasführung 901 ausleitet und beispielsweise zurück in die Sterilisationsvorrichtung 20. In der Sterilisationsvorrichtung 20 ist in manchen Ausführungsformen eine Abgasaufbereitung vorgesehen. Die Abgasaufbereitung kann beispielsweise einen Filter, etwa mit Aktivkohle bestückt, umfassen, welcher den plasmaaktivierten Wasserdampf entsprechend reinigt. Dazu kann in manchen Ausführungsformen auch eine Verwendung eines Katalysators angedacht sein, welcher die langlebigen, aktiven Spezies umwandelt. Alternativ oder ergänzend kann auch ein zweiter Plasmagenerator vorgesehen sein, durch welchen der plasmaaktivierte Wasserdampf geleitet wird, also als Arbeitsgas verwendet wird, um die langlebigen Spezies in kurzlebige Spezies zu überführen. In manchen Ausführungsformen wird durch den zweiten Plasmagenerator ein Plasmastrom erzeugt, durch welchen das aus der Patientengasführung 901 als Abluft austretende plasmaaktivierte Wasserdampf geleitet wird. Die durch die erneute Plasmabehandlung entstehenden kurzlebigen Spezies zerfallen innerhalb einer kurzen Zeit (etwa unter 60 Sekunden).

Alternativ oder ergänzend kann auch eine Anordnung einer Absaugeinrichtung in der Sterilisationsvorrichtung 20 realisiert werden, um den plasmaaktivierten Wasserdampf durch die Patientengasführung 901 zu fördern. Die Sterilisationsvorrichtung 20 wird dabei so an die Patientengasführung 901 angeschlossen, dass die Absaugung beispielhaft über den Inspirationsanschluss 9011 den plasmaaktivierten Wasserdampf durch die Patientengasführung 901 saugen kann. Die Abgasaufbereitung ist in bei einer Ausführungsform mit Absaugeinrichtung beispielsweise so angeordnet, dass der plasmaaktivierte Wasserdampf bzw. das Abgas, welches über den Inspirationsanschluss 9011 aus der Patientengasführung 901 ausgeleitet wird, durch die Abgaseinrichtung, umfassend zum Beispiel eine Filtereinrichtung und/oder eine Plasmabehandlung, gezogen wird.

In manchen Ausführungsformen umfasst die Sterilisationseinrichtung 20 eine eigene Fördereinheit, welche den plasmaaktivierten Wasserdampf durch die Patientengasführung 901 fördert. In manchen Ausführungsformen ist die Fördereinheit 103 des Beatmungsgerätes 10 so eingerichtet, dass auch diese mit dem plasmaaktivierten Wasserdampf aufbereitet werden kann ohne Schaden zu nehmen. In einem solchen Fall kann die Förderung des plasmaaktivierten Wasserdampfs durch die Patientengasführung 901 auch durch die Fördereinheit 103 geleistet werden.

Es können insbesondere zwei Sterilisationsmodi angedacht sein. In einem ersten Modus wird die Patientengasführung 901 pulsweise mit plasmaaktiviertem Wasserdampf durchspült. Beispielsweise werden die Ventile 9003 und 9004 dazu jeweils kurz geöffnet, dass der plasmaaktivierte Wasserdampf in die Patientengasführung 901 strömen kann. Für eine gewissen, optional einstellbare, Zeit werden die Ventile 9003 und 9004 wieder geschlossen, sodass der plasmaaktivierte Wasserdampf in der Patientengasführung 901 stehen und etwas einwirken kann. Durch Öffnen der Ventile 9003 und 9004 kann frischer plasmaaktivierter Wasserdampf nachströmen. Dieser Zyklus kann mehrfach, beispielsweise über einen Zeitraum von 10 bis 60 Minuten oder länger wiederholt werden.

In einem anderen Sterilisationsmodus ist eine dauerhafte Durchspülung der Patientengasführung mit plasmaaktiviertem Wasserdampf vorgesehen. Dazu sind die Ventile 9003 und 9004 dauerhaft geöffnet und der plasmaaktivierte Wasserdampf wird kontinuierlich durch die Patientengasführung 901 gefördert.

In manchen Ausführungsformen kann vorgesehen sein, dass zum Abschluss der Sterilisation zumindest die Patientengasführung 901 mit Gas gespült wird. Beispielsweise kann dazu von der Sterilisationsvorrichtung 20 das Arbeitsgas durch die Patientengasführung 901 gefördert werden, wobei der Plasmagenerator nicht aktiv ist und auch keine Wasser und/oder Wasserdampf eingespeist wird. In manchen Ausführungsformen ist es auch angedacht, dass alternativ oder ergänzend Frischgas aus der Atemgasquelle 102 durch die Patientengasführung 901 geleitet wird.

Die Sterilisationsvorrichtung 20 verfügt beispielhaft über eine eigene Energieversorgung, Gasquellen und Wasserquellen. Zudem kann die Sterilisationsvorrichtung 20 über Steuereinheiten verfügen, welche dazu ausgebildet sind, die Erzeugung und/oder Förderung des plasmaaktivierten Wasserdampfs zu steuern. Es ist zudem vorgesehen, dass über eine Benutzerschnittstelle Eingaben zur Steuerung der Sterilisationsvorrichtung 20 gemacht werden können. Zumindest kann die Sterilisationsvorrichtung 20 über eine Benutzerschnittstelle ein- und ausgeschaltete bzw. aktiviert und deaktiviert werden.

In manchen Ausführungsformen sind das Beatmungsgerät 10 und die Sterilisationsvorrichtung 20 über eine Schnittstelle miteinander verbunden. Beispielhaft kann so eine Steuerung der Sterilisationsvorrichtung 20 über das Beatmungsgerät 10 realisiert werden. Alternativ oder ergänzend kann über eine Verbindung zwischen dem Beatmungsgerät 10 und der Sterilisationsvorrichtung 20 auch eine zumindest teilweise Steuerung der Ventile des Beatmungsgerätes 10 durch die Sterilisationsvorrichtung 20 erreicht werden.

In manchen Ausführungsformen ist es vorgesehen, dass die Sterilisationsvorrichtung 20 neben einer Verbindung zur Steuerung durch das Beatmungsgerät 10 auch eine Energieversorgung und/oder eine Gasversorgung und/oder eine Wasserversorgung von dem Beatmungsgerät 10 erhält. In dem Beatmungsgerät 10 kann auch eine Absaugeinheit 110 (in Figur 1 nicht dargestellt) vorgesehen sein, welche zur Förderung des plasmaaktivierten Wasserdampfes durch die Patientengasführung 901 genutzt wird. Beispielsweise ist die Absaugeinheit 110 direkt mit der Patientengasführung 901 verbunden. Es kann auch vorgesehen sein, dass die Absaugeinheit 110 einen zusätzlichen Anschluss umfasst, über welchen der Inspirationsanschluss 9011 mit der Absaugeinheit 110 während der Sterilisation verbunden wird.

In manchen Ausführungsformen ist vorgesehen, dass die Sterilisationsvorrichtung 20 Teil des Beatmungsgerätes 10 ist. Beispielweise ist die Sterilisationsvorrichtung 20 in das Gehäuse 101 des Beatmungsgerätes 10 integriert, sodass in dem Gehäuse 101 sowohl die Komponenten des Beatmungsgerätes 10 als auch die Sterilisationsvorrichtung 20 angeordnet sind. Die Sterilisationsvorrichtung 20 ist dann beispielsweise so angeordnet, dass über zumindest einen Anschluss die Sterilisationsvorrichtung 20 mit dem Exspirationsanschluss 9012 verbunden werden kann und so plasmaaktivierter Wasserdampf in die Patientengasführung 901 geleitet wird. Ist die Sterilisationsvorrichtung 20 in das Beatmungsgerät 10 integriert, kann beispielsweise auch die vollständige Steuerung der Sterilisation über das Beatmungsgerät 10 bzw. die Benutzerschnittstelle 107 des Beatmungsgerätes 10 erfolgen.

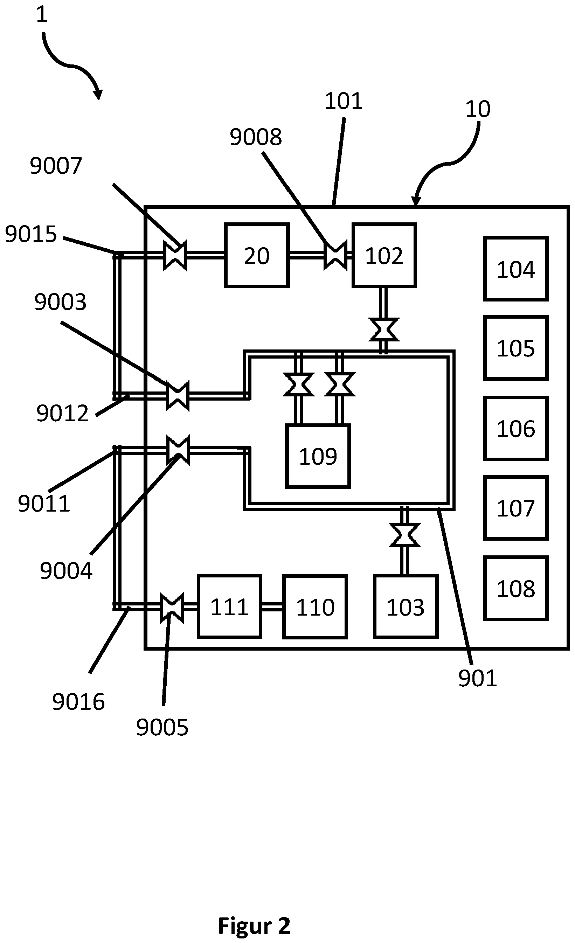

In Figur 2 ist eine beispielhafte Ausführungsform des Systems 1 schematisch dargestellt, wobei das Beatmungsgerät 10 über eine integrierte Absaugeinheit 110 verfügt und die Sterilisationsvorrichtung 20 in das Beatmungsgerät 10 integriert ist. Das Beatmungsgerät 10 ist, analog zu der in Figur 1 dargestellten Ausführungsform, als Anästhesiegerät ausgeführt und verfügt, soweit nicht explizit anders beschrieben, zumindest über den gleichen Funktionsumfang. Im Allgemeinen handelt es sich um ein Anästhesiegerät gemäß dem Stand der Technik, wobei die erfindungsgemäßen Unterschiede im Folgenden beschrieben werden.

Die Sterilisationsvorrichtung 20 ist dazu eingerichtet und ausgebildet plasmaaktivierten Wasserdampf zu erzeugen. Das Arbeitsgas zur Erzeugung des kalten Plasmas in der Sterilisationsvorrichtung wird beispielhaft durch die Atemgasquelle 102 geliefert. In manchen Ausführungsformen umfasst die Atemgasquelle 102 auch eine Quelle für Anästhesiegas, wobei zur Sterilisation kein Anästhesiegas zur Sterilisationsvorrichtung 20 geleitet wird. Die Förderung von Gas von der Atemgasquelle 102 zur Sterilisationsvorrichtung 20 kann beispielsweise durch das Ventil 9008 gesteuert werden, zumindest kann über das Ventil 9008 gesteuert werde, ob Gas von der Atemgasquelle 102 zur Sterilisationsvorrichtung 20 geleitet wird. In manchen Ausführungsformen verfügt die Sterilisationsvorrichtung 20 über einen von der Atemgasquelle 102 unabhängigen Gasanschluss. Die Wasserversorgung der Sterilisationsvorrichtung 20 wird beispielsweise über einen in das Beatmungsgerät 10 beziehungsweise in die Sterilisationsvorrichtung 20 integrierten Wassertank und/oder durch einen Anschluss zu einer externen Wasserversorgung erreicht.

Zur Sterilisation wird der von der Sterilisationsvorrichtung 20 ausgehende Sterilisationsanschluss 9015 mit dem Exspirationsanschluss 9012 verbunden. Durch diese Verbindung kann, bei geöffneten Ventilen 9007 und 9003, der plasmaaktivierte Wasserdampf in die Patientengasführung 901 geleitet werden.

Die Absaugeinheit 110 des Beatmungsgerätes 10 ist unter anderem dazu eingerichtet, zur Absaugung von Sekret, beispielsweise aus den Atemwegen eines Patienten, verwendet zu werden. Dazu wird an den Absauganschluss 9016 ein entsprechender Auffangbehälter angeschlossen, in welchen das Sekret gesaugt wird. Beispielsweise ist zwischen Absauganschluss 9016 und Absaugeinheit 110 ein Ventil 9005 angeordnet, über welches die Absaugung zumindest teilweise kontrolliert werden kann. Die Absaugeinheit 110 umfasst beispielhaft eine Vakuumpumpe, mit welcher ein Unterdruck erzeugt werden kann, welcher zur Absaugung genutzt wird.

Während der Sterilisation kann die Absaugeinheit 110 dazu genutzt werden den plasmaaktivierten Wasserdampf durch die Patientengasführung 901 zu fördern. Dazu wird der Absauganschluss 9016 mit dem Inspirationsanschluss 9011 verbunden. In der in Figur 2 beispielhaft dargestellten Ausführungsform ist zwischen dem Absauganschluss 9016 und der Absaugeinheit 110 eine Abgasbehandlung 111 angeordnet. Durch die Abgasbehandlung 111 wird der plasmaaktivierte Wasserdampf geleitet, nachdem er durch die Patientengasführung 901 geleitet wurde. In der Abgasbehandlung 111, beispielsweise als Filter und/oder Plasmagenerator ausgeführt, wird der plasmaaktivierte Wasserdampf dahingehend aufbereitet, dass zumindest ein Teil der langlebigen aktiven Spezies herausgefiltert oder in kurzlebige Spezies, welche im Mittel in einer Zeit <60 Sekunden zerfallen, umgewandelt werden. Als Filtermaterial kann beispielsweise Aktivkohle dienen. Die Abgasbehandlung 111 kann beispielsweise auch dazu eingesetzt werden, das Gas, welches mit der Absaugung von Sekret durch die Absaugeinheit 110 angesogen wird, aufzubereiten bevor es in die Umgebungsluft abgegeben wird. In manchen Ausführungsformen verfügt die Abgasbehandlung 111 einen Bypass, sodass nur während der Sterilisation Gas bzw. der plasmaaktivierte Wasserdampf, durch die Abgasbehandlung 111 aufbereitet wird.

Durch die Integration der Absaugeinheit 110 in das Beatmungsgerät 10 kann die in der in Figur 1 dargestellten Ausführungsform optional in der Sterilisationsvorrichtung 20 angeordnete Absaugeinheit eingespart werden.

Gesteuert wird die Sterilisation beispielsweise über die Steuereinheit 104. Es ist vorgesehen, dass über die Benutzerschnittstelle 107 Eingaben zur Sterilisation möglich sind, beispielsweise die Dauer und/oder einen Modus betreffend. In manchen Ausführungsformen kann auch vorgesehen sein, dass der Fluss und/oder eine mittlere Verweilzeit des plasmaaktivierten Wasserdampfs in der Patientengasführung 901 einstellbar ist. Auch die Menge an in den Plasmastrom injiziertem Wasser bzw. Wasserdampf kann einstellbar sein. Über die Steuereinheit 104 werden beispielhaft die Ventile des Beatmungsgerätes 10 so geschaltet, dass der plasmaaktivierte Wasserdampf ausschließlich durch die Patientengasführung 901 gefördert wird. Auch wird über die Steuereinheit 104 die Zufuhr von Arbeitsgas zur Sterilisationsvorrichtung 20 gesteuert. In manchen Ausführungsformen sind die Sensoreinheit 105 und/oder die Erkennungseinheit 108 so ausgebildet und eingerichtet, dass die Sterilisation überwacht wird. Beispielsweise kann vorgesehen sein, dass bei einer erkannten Leckage, durch welche plasmaaktivierter Wasserdampf aus dem System 1 und/oder dem Beatmungsgerät 10 austreten kann die Sterilisation unterbrochen wird und gegebenenfalls ein Alarm generiert wird.

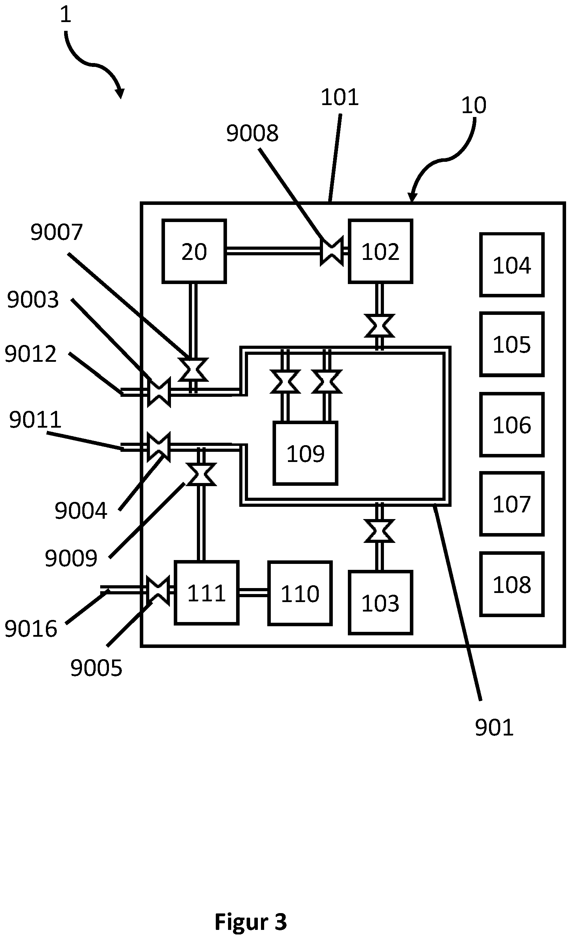

Figur 3 zeigt eine schematische Darstellung einer beispielhaften Ausführungsform des Systems 1, bei der das Beatmungsgerät 10 als Anästhesiegerät ausgeführt ist und die Sterilisationsvorrichtung 20 sowie eine Absaugeinheit 110 in das Beatmungsgerät 10 integriert sind. Die wesentlichen Funktionsmerkmale des Beatmungsgerätes 10 entsprechen dabei einem Anästhesiegerät nach dem Stand der Technik beziehungsweise wie zu Figur 1 und/oder 2 beschrieben.

Die in das Beatmungsgerät 10 integrierte Sterilisationsvorrichtung 20 ist dazu eingerichtet und ausgebildet unter Generation eines kalten Plasmas plasmaaktivierten Wasserdampf zu erzeugen. Beispielsweise wird das Arbeitsgas, welches der Plasmagenerator der Sterilisationsvorrichtung 20 zur Generation des kalten Plasmas verwendet, durch die Atemgasquelle 102 bereitgestellt. Ergänzend oder alternativ kann die Sterilisationsvorrichtung 20 über eine extern angeschlossene Gasquelle, beispielsweise Druckgasflaschen, direkt und unabhängig von der Atemgasquelle 102 mit dem Arbeitsgas versorgt werden. Ein in das Beatmungsgerät 10 beziehungsweise die Sterilisationsvorrichtung 20 integrierte Wassertank und/oder Anschluss für eine externe Wasserquelle ist dafür vorgesehen, die Sterilisationsvorrichtung 20 mit Wasser zu versorgen.

Während der Beatmung eines Patienten sind das Ventil 9008 zwischen Atemgasquelle 102 und Sterilisationsvorrichtung 20 sowie das Ventil 9007 zwischen Sterilisationsvorrichtung 20 und Patientengasführung 901 geschlossen. Zur Sterilisation werden beide Ventile 9007, 9008 geöffnet, damit Arbeitsgas zur Sterilisationsvorrichtung 20 und von dort plasmaaktivierter Wasserdampf in die Patientengasführung 901 geleitet werden kann. In manchen Ausführungsformen werden die Ventile zwischen der Atemgasquelle 102, der Fördereinheit 103 sowie dem CO2-Absorber 109 und der Patientengasführung 901 während der Sterilisation geschlossen. In manchen Ausführungsformen ist zumindest eins von Atemgasquelle 102, Fördereinheit 103 und/oder CO2-Absorber 109 stabil gegenüber dem plasmaaktivierten Wasserdampf und kann mit in den Sterilisationslauf eingebunden werden.

Im Unterschied zu der in Figur 2 dargestellten Ausführungsform ist die Sterilisationsvorrichtung 20 innerhalb des Beatmungsgerätes 10 direkt mit der Patientengasführung 901 verbunden und wird nicht über außerhalb des Gehäuses 101 gelegene Anschlüsse mit dem Exspirationsanschluss 9012 verbunden. Entsprechend ist das Ventil 9003 während der Sterilisation geschlossen, damit der plasmaaktivierte Wasserdampf nicht ungewollt aus dem Exspirationsanschluss 9012 in die Umgebungsluft entweichen kann. In manchen Ausführungsformen kann der Exspirationsanschluss 9012 beispielsweise durch einen Blindstopfen verschlossen werden.

In das Beatmungsgerät 10 ist weiter eine Absaugeinheit 110, beispielsweise eine Vakuumpumpe, angeordnet. Die Absaugeinheit 110 ist unter anderem dafür vorgesehen, plasmaaktivierten Wasserdampf durch die Patientengasführung 901 zu fördern bzw. zu saugen. Zwischen Patientengasführung 901 und Absaugeinrichtung 110 ist eine Abgasbehandlung 111, beispielsweise ein Filter und/oder ein Plasmagenerator, angeordnet, welcher unter anderem dafür vorgesehen ist, den plasmaaktivierten Wasserdampf, welcher durch die Patientengasführung 901 geleitet wurde, aufzubereiten. In diesem Zusammenhang bedeutet eine Aufbereitung, dass langlebige, aktive Spezies herausgefiltert und/oder in kurzlebige Spezies umgewandelt werden, welche im Mittel innerhalb <60 Sekunden zerfallen. Die Absaugeinheit 110 ist direkt mit der Patientengasführung 901 verbunden, sodass eine Verbindung zwischen dem Inspirationsanschluss 9011 und einem Absauganschluss nicht extra hergestellt werden muss. Ist die Absaugeinheit 110 direkt mit der Patientengasführung verbunden, wird das Ventil 9004 an dem Inspirationsanschluss 9011 während der Sterilisation verschlossen.

In dem Beatmungsgerät 10 ist zusätzlich ein Absauganschluss 9016 vorgesehen, welcher zu der Absaugeinheit 110 führt. Beispielhaft läuft die Verbindung zwischen Absauganschluss 9016 und Absaugeinheit 110 über die Abgasbehandlung 111. In manchen Ausführungsformen kann aber auch ein Bypass vorgesehen sein und/oder die Abgasbehandlung 111 so angeordnet sein, dass der Absauganschluss 9016 direkt mit der Absaugeinheit 110 verbunden ist. Der Absauganschluss 9016 dient beispielsweise dem Anschluss eines Auffangbehälters bei der Verwendung der Absaugeinheit 110 zum Absaugen von Sekret des Patienten während der Beatmung. Es kann zudem auch vorgesehen sein, dass die Absaugeinheit 110 nicht innerhalb des Beatmungsgerätes 10 mit der Patientengasführung 901 verbunden ist. In einem solchen Fall ist die Verbindung des Absauganschlusses 9016 mit dem Inspirationsanschluss 9011 vorgesehen. Während der Sterilisation wird dann über den Inspirationsanschluss 9011 bei geöffnetem Ventil 9004 der plasmaaktivierte Wasserdampf durch die Patientengasführung 901 gesaugt.

Die Verbindung zwischen der Sterilisationsvorrichtung 20 und der Patientengasführung 901 sowie die Verbindung zwischen der Absaugeinheit 110 und der Patientengasführung 901 sind beispielhaft so angeordnet, dass der plasmaaktivierte Wasserdampf die gesamte Patientengasführung 901 durchläuft. Dazu ist die Sterilisationsvorrichtung 20 beispielhaft in unmittelbarer Nähe zum Exspirationsanschluss 9012 mit der Patientengasführung 901 verbunden und die Absaugeinheit 110 ist in unmittelbarer Nähe zum Inspirationsanschluss 9011 mit der Patientengasführung 901 verbunden bzw. über den Inspirationsanschluss 9011 mit der Patientengasführung 901 verbunden.

Gesteuert wird die Sterilisation beispielsweise über die Steuereinheit 104. Es ist vorgesehen, dass über die Benutzerschnittstelle 107 Eingaben zur Sterilisation möglich sind, beispielsweise die Dauer und/oder einen Modus betreffend. In manchen Ausführungsformen kann auch vorgesehen sein, dass der Fluss und/oder eine mittlere Verweilzeit des plasmaaktivierten Wasserdampfs in der Patientengasführung 901 einstellbar ist. Auch die Menge an in den Plasmastrom injiziertem Wasser bzw. Wasserdampf kann einstellbar sein. Über die Steuereinheit 104 werden beispielhaft die Ventile des Beatmungsgerätes 10 so geschaltet, dass der plasmaaktivierte Wasserdampf ausschließlich durch die Patientengasführung 901 gefördert wird. Auch wird über die Steuereinheit 104 die Zufuhr von Arbeitsgas zur Sterilisationsvorrichtung 20 gesteuert. In manchen Ausführungsformen sind die Sensoreinheit 105 und/oder die Erkennungseinheit 108 so ausgebildet und eingerichtet, dass die Sterilisation überwacht wird. Beispielsweise kann vorgesehen sein, dass bei einer erkannten Leckage, durch welche plasmaaktivierter Wasserdampf aus dem System 1 und/oder dem Beatmungsgerät 10 austreten kann die Sterilisation unterbrochen wird und gegebenenfalls ein Alarm generiert wird.

In manchen Ausführungsformen verfügt das Beatmungsgerät 10 nicht über eine Absaugeinheit 110. Bei solchen Ausführungsformen ist daran gedacht, dass beispielsweise eine Abgasbehandlung 111 in das Beatmungsgerät 10 integriert ist und/oder über den Inspirationsanschluss 9011 mit der Patientengasführung 901 verbindbar ist. In manchen Ausführungsformen ist angedacht, dass eine Absaugeinheit extern des Beatmungsgerätes 110 mit dem Inspirationsanschluss 9011 verbunden wird, um den plasmaaktivierten Wasserdampf durch die Patientengasführung 901 zu fördern bzw. bei der Förderung zu unterstützen.

Insbesondere wenn keine Absaugeinheit 110 in dem Beatmungsgerät 10 integriert ist, ist vorgesehen, dass der plasmaaktivierte Wasserdampf durch den Druck des Arbeitsgases der Sterilisationsvorrichtung 20 durch die Patientengasführung 901 gefördert wird. Alternativ oder ergänzend ist angedacht, dass die Förderung des plasmaaktivierten Wasserdampfs durch die Fördereinheit 103 und/oder eine zusätzliche Fördereinheit nur für die Sterilisation, etwa ein Gebläse, zumindest unterstütz wird.

Nach Beendigung der Sterilisation ist in manchen Ausführungsformen vorgesehen, dass für einige Zeit Arbeitsgas und/oder Frischgas (beispielsweise Atemgas aus der Atemgasquelle 102) durch die Patientengasführung 901 gefördert wird um mögliche Rückstände des plasmaaktivierten Wasserdampfs, etwa Ozon, aus der Patientengasführung 901 zu spülen.

Die interne Verbindung der Sterilisationsvorrichtung 20 mit der Patientengasführung 901 ermöglicht in manchen Ausführungsformen eine automatische Sterilisation der Patientengasführung 901. Insbesondere entfällt der Schritt die Sterilisationsvorrichtung 20 mit der Patientengasführung 901 zu verbinden. Bei der automatischen Sterilisation ist die Steuereinheit 104 dazu eingerichtet, die Ventile des Beatmungsgerätes 10 so zu schalten, dass der, von der Sterilisationsvorrichtung 20 erzeugte, plasmaaktivierte Wasserdampf durch die Patientengasführung 901 gefördert werden kann. Entsprechend der über die Benutzerschnittstell 107 vorgegebenen Einstellungen bezüglich der Sterilisation, wird die Förderung und Erzeugung des plasmaaktivierten Wasserdampfs gesteuert. Sofern die Benutzerschnittstelle 107 bzw. das System 1 über eine Anzeige verfügt, kann vorgesehen sein, einen Status der Sterilisation anzuzeigen, zumindest ob die Sterilisation derzeit durchgeführt wird und/oder ob die Sterilisation abgeschlossen ist.

Die automatische Sterilisation kann beispielsweise so ablaufen, dass nach der Aktivierung zunächst geprüft wird, ob die Ventile so geschaltet sind, dass der plasmaaktivierte Wasserdampf ausschließlich durch die Patientengasführung 901 und/oder die zur Sterilisation vorgesehenen Komponenten geleitet wird. Darauffolgend wird die Sterilisationsvorrichtung 20 aktiviert und der plasmaaktivierte Wasserdampf wird erzeugt. Je nach Einstellung wird beispielsweise ein dauerhafter Strom an plasmaaktiviertem Wasserdampf oder ein gepulster Strom an plasmaaktiviertem Wasserdampf durch die Patientengasführung 901 gefördert. Die Dauer der Sterilisation ist beispielsweise über die Benutzerschnittstelle 107 zu wählen. Nach Beendigung der Sterilisation im Sinne der Erzeugung und Förderung des plasmaaktivierten Wasserdampfes kann vorgesehen sein, dass für eine definierbare Dauer Frischgas und/oder Arbeitsgas durch die Patientengasführung 901 gefördert wird.

Eine beispielhafte Ausführungsform des Systems 1, bei dem das Beatmungsgerät 10 als Heimbeatmungsgerät, beispielsweise zur lebenserhaltenen Beatmung und/oder Schlaftherapie, bzw. als Beatmungsgerät zur klinischen Beatmung ausgebildet ist. Das Beatmungsgerät 10 entspricht dabei im Wesentlichen einem Gerät nach dem Stand der Technik und verfügt zumindest über eine Atemgasquelle 102, eine Fördereinheit 103 sowie eine Steuereinheit 104, eine Sensoreinheit 105, eine Auswerteeinheit 106, eine Benutzerschnittstelle 107 sowie eine Erkennungseinheit 108. Die Atemgasquelle 102 und die Fördereinheit 103 können beispielsweise als eine Einheit ausgeführt sein, beispielsweise als Gebläse, welches Umgebungsluft ansaugt und durch die Gasführung, beispielsweise den Inspirationsweg 9018, zum Patienten fördert.

Die Steuereinheit 104 ist dazu eingerichtet zumindest die Atemgasquelle 102 und die Fördereinheit 103 zu steuern. Die Steuereinheit 104 kann beispielsweise dazu in mehrere Steuereinheiten unterteilt sein, welche jeweils zur Steuerung einer Komponente des Beatmungsgerätes 10 und/oder des Systems 1 eingerichtet sind. In manchen Ausführungsformen ist die Steuereinheit 104 dazu eingerichtet auch die Sterilisationsvorrichtung 20 zu steuern. Die Steuereinheit 104 ist beispielhaft auch dazu eingerichtet, Werte, Daten und Informationen von der Sensoreinheit 105, der Auswerteeinheit 106, der Benutzerschnittstelle 107 und/oder der Erkennungseinheit 108 zu verarbeiten und/oder als Grundlage der Steuerung der Komponenten des Beatmungsgerätes 10 und/oder des Systems 1 zu nutzen. Ist eine Steuerung der Sterilisationsvorrichtung 20 über das Beatmungsgerät 10 vorgesehen, werden das Beatmungsgerät 10 und die Sterilisationsvorrichtung 20 beispielsweise über eine Schnittstelle miteinander verbunden.

Die Sensoreinheit 105 ist beispielsweise dazu eingerichtet um über mit ihr verbundene Sensoren Messwerte und Messdaten zu erfassen, welche beispielsweise in dem Beatmungsgerät 10 oder allgemein in dem System 1 angeordnet sind. Diese Messwerte und Messdaten können beispielsweise die Beatmung des Patienten, wie zum Beispiel Fluss, Druck, Gaszusammensetzung, Temperatur, Konzentration des Blutsauerstoffs, Herzschlag und/oder Feuchtigkeit umfassen. Auch weitere, optional auch nicht direkt mit der Beatmung zusammenhängende Daten und Werte, können beispielhaft über die Sensoreinheit 105 erfasst werden.

Die Auswerteeinheit 106 ist beispielhaft dazu ausgebildet, unter anderem die Messwerte und/oder Messdaten der Sensoreinheit 105 und/oder anderer Datenquellen, wie zum Beispiel über eine Benutzerschnittstelle 107 eingegebene Daten und Informationen, aufzubereiten, weiterzuverarbeiten, zu analysieren und/oder zu bewerten.

Im System 1 bzw. beispielhaft im Beatmungsgerät 10 ist eine Benutzerschnittstelle 107 angeordnet, welche zumindest eine Eingabevorrichtung und/oder Anzeigevorrichtung umfasst. Über die Eingabevorrichtung, beispielsweise eine Tastatur und/oder (Dreh-)Knöpfe und/oder eine berührungsempfindliche Oberfläche (Touchscreen) können Daten, Informationen und Werte eingegeben werden und auch Vorgaben zur Steuerung des Beatmungsgerätes 10 und/oder des Systems 1. Eine Anzeigevorrichtung kann beispielsweise ein Display sein oder auch eine analoge Anzeige oder auch einfache Leuchtsymbole. Die Anzeigeeinrichtung ist beispielsweise dazu eingerichtet, Daten und/oder Werte und/oder Informationen anzuzeigen, welche unter anderem den Status des Systems 1 und/oder die Beatmung eines Patienten betreffen. In manchen Ausführungsformen sind über die Benutzerschnittstelle 107 zudem Eingaben zur Steuerung der Sterilisationsvorrichtung 20 möglich.

Die Erkennungseinheit 108 ist in beispielhaft dazu eingerichtet, technische Probleme des Beatmungsgerätes 10 zu erfassen. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Auswerteeinheit 108 kann bei einem erkannten technischen Problem einen Alarm auf dem Beatmungsgerät 10 und/oder über eine Schnittstelle (nicht dargestellt) anzeigen und/oder übermitteln. In manchen Ausführungsformen ist die Erkennungseinheit 107 auch dazu eingerichtet technische Probleme des gesamten Systems 1 zu erfassen, beispielsweise einer extern des Beatmungsgerätes 10 angeordneten Sterilisationsvorrichtung 20, welche beispielsweise mit dem Beatmungsgerät 10 verbunden ist.

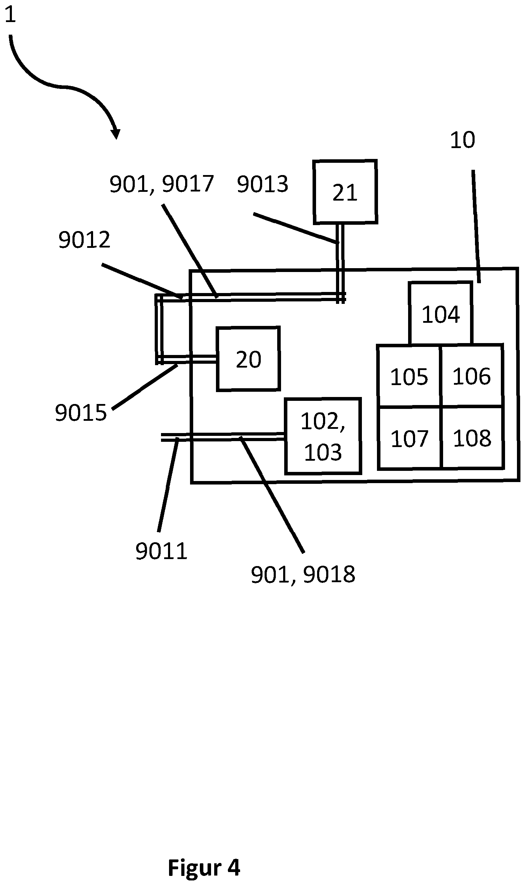

Das in Figur 4 beispielhaft dargestellte Beatmungsgerät 10 verfügt über zwei im Wesentlichen voneinander getrennte Gasführungen (Exspirationsweg 9017 und Inspirationsweg 9018). Über den Inspirationsweg 9018 wird während der Beatmung zumindest zeitweise Atemgas von der Atemgasquelle 102 zum Patienten gefördert. Beispielhaft ist das Beatmungsgerät 10 für eine Beatmung über ein Zwei-Schlauch-System eingerichtet, wobei die vom Patienten ausgeatmete Luft durch einen Schlauch vom Patienten zum Exspirationsanschluss 9012 und durch den Exspirationsweg 9017 im Beatmungsgerät 10 zur Abluft 9013 geleitet wird.

Das beispielhaft dargestellte Beatmungsgerät 10 umfasst weiter auch eine Sterilisationsvorrichtung 20 zur Erzeugung von plasmaaktiviertem Wasserdampf zur zumindest teilweisen Sterilisation des Beatmungsgerätes 10. Insbesondere ist vorgesehen, dass der Exspirationsweg 9017 des Beatmungsgerätes 10 durch die Sterilisationsvorrichtung 20 hygienisch aufbereitet werden kann. Zur Aufbereitung wird dazu der Sterilisationsanschluss 9015 mit dem Exspirationsanschluss 9012 verbunden. Beispielsweise über die Sterilisationsvorrichtung 20 wird der plasmaaktivierte Wasserdampf durch den Exspirationsweg 9017 gefördert und reinigt diesen hygienisch auf. Der plasmaaktivierte Wasserdampf kann über die Abluft 9013 aus dem Exspirationsweg 9017 ausgeleitete werden. In manchen Ausführungsformen ist es nötig den plasmaaktivierten Wasserdampf nach der Abluft 9013 aufzubereiten. Beispielsweise kann dazu eine Abgasbehandlung 21 an die Abluft 9013 angeschlossen werden. Die Abgasbehandlung 21 umfasst beispielsweise einen Filter und/oder einen Plasmagenerator um die langlebigen aktiven Spezies, etwa Ozon, aus dem plasmaaktivierten Wasserdampf zu filtern bzw. umzuwandeln und/oder zu neutralisieren. In manchen Ausführungsformen kann die Abgasbehandlung 21 auch in das Beatmungsgerät 10 integriert sein.

Die Versorgung der Sterilisationsvorrichtung 20 mit Arbeitsgas kann beispielsweise über extra Gasquellen, etwa Druckgasflaschen, und/oder über die Atemgasquelle 102 erfolgen. Beispielsweise kann dazu vorgesehen sein, dass die Sterilisationsvorrichtung 20 einen Gaseingang aufweist, welcher mit dem Inspirationsanschluss 9011 verbunden wird.

Es ist auch daran gedacht, den Inspirationsweg 9018 mit in die Sterilisation einzubeziehen. Beispielsweise wird dazu innerhalb des Beatmungsgerätes 10 die Sterilisationsvorrichtung 20 mit dem Inspirationsweg 9018 verbunden und der Inspirationsanschluss 9011 mit dem Exspirationsanschluss 9012. Somit durchströmt der plasmaaktivierte Wasserdampf zunächst den Inspirationsweg 9018, wird dann durch den Inspirationsanschluss 9011 zum Exspirationsanschluss 9012 geleitet und durchströmt schließlich den Exspirationsweg 9017 bis zur Abluft 9013.

In manchen Ausführungsformen des Systems 1 ist es angedacht, dass eine in das Beatmungsgerät 10 integrierte Sterilisationsvorrichtung 20 auch zur Sterilisation anderer Geräte nutzbar ist, insbesondere solcher, welche von einem plasmaaktivierten Wasserdampf durchströmt werden können.

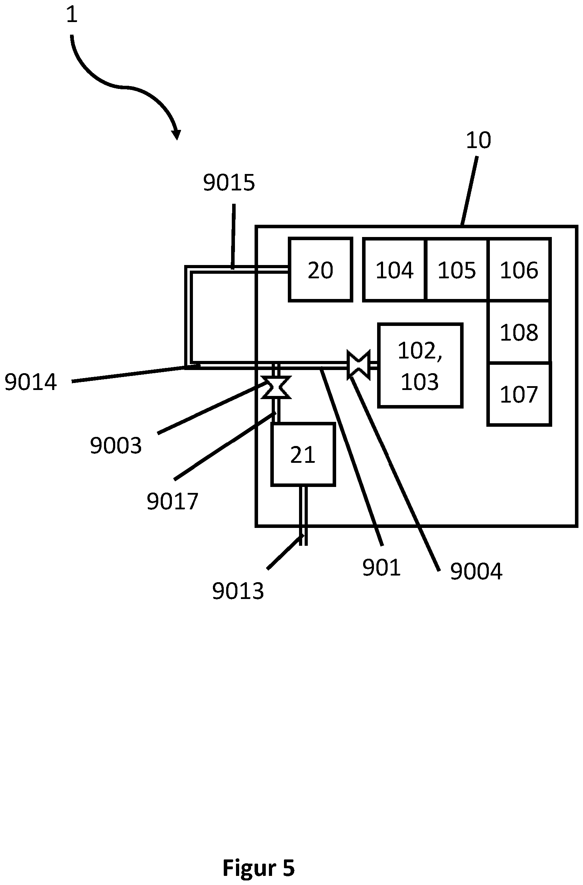

Eine weitere beispielhafte Ausführungsform des Systems 1 ist in Figur 5 dargestellt. Das Beatmungsgerät 10 ist, ähnlich wie bei der Ausführungsform in Figur 4, als Heimbeatmungsgerät bzw. Beatmungsgerät zur klinischen Beatmung ausgeführt. Das dargestellte Beatmungsgerät 10 ist beispielhaft für einen Betrieb in einem Ein-Schlauch-System, beispielsweise mit Leckage-System oder Ventil-System) eingerichtet. Das Patienteninterface wird für die Beatmung über den Patientenanschluss 9014 mit der Patientengasführung 901 des Beatmungsgerätes 10 verbunden. Die Patientengasführung 901 teilt sich dabei in einen Inspirationsweg (nicht gekennzeichnet) und einen Exspirationsweg 9017 auf. Über die Ventile 9003, 9004 kann während der Beatmung der Atemgasfluss zur Inspiration und Exspiration gesteuert werden. Beispielsweise wird während der Inspiration das Ventil 9004 geöffnet und das Ventil 9003 geschlossen. Dadurch wird Atemgas durch den Patientenanschluss 9014 zum Patienten gefördert. Während der Exspiration des Patienten wird das Ventil 9004 zumindest teilweise geschlossen und das Ventil 9003 geöffnet, sodass das zurückströmende Atemgas durch den Exspirationsweg 9017 entweichen kann.

Zur Sterilisation des Beatmungsgerätes 10 ist in das Beatmungsgerät 10 eine Sterilisationsvorrichtung 20 integriert. Die Sterilisationsvorrichtung 20 ist dazu ausgebildet und eingerichtet plasmaaktivierten Wasserdampf zu erzeugen und zumindest teilweise zu fördern. Vor Beginn der Sterilisation wird die Sterilisationsvorrichtung 20 durch eine Verbindung zwischen dem Sterilisationsanschluss 9015 und dem Patientenanschluss 9014 zumindest mit Teilen der Patientengasführung 901 verbunden. Es ist vorgesehen, dass zumindest der Exspirationsweg 9017 der Patientengasführung 901 mit plasmaaktiviertem Wasserdampf durchströmt werden kann. Beispielsweise ist die Sterilisationsvorrichtung 20 dazu eingerichtet den plasmaaktivierten Wasserdampf zumindest teilweise durch die Patientengasführung 901 zu fördern. Damit der Exspirationsweg 9017 hygienisch aufbereitet bzw. sterilisiert wird, wird zur Sterilisation das Ventil 9003 geöffnet. Dadurch wird ermöglicht, dass der plasmaaktivierte Wasserdampf durch den Exspirationsweg 9017 strömt.

Um zu verhindern, dass die langlebigen aktiven Spezies des plasmaaktivierten Wasserdampfs in die Umgebungsluft abgegeben werden, ist nach dem Exspirationsweg 9017 beispielhaft eine Abgasbehandlung 21 angeordnet. Die Abgasbehandlung 21 umfasst beispielsweise einen Filter, welcher die aktiven Spezies aus dem plasmaaktivierten Wasserdampf, welcher zumindest teilweise durch die Patientengasführung 901 geleitet wurde, herausfiltert. In manchen Ausführungsformen ist umfasst die Abgasbehandlung 21 alternativ oder ergänzend einen Plasmagenerator, welcher durch ein Plasma die langlebigen Spezies des plasmaaktivierten Wasserdampfs in kurzlebigere Spezies umwandelt, welche im Mittel innerhalb einer Zeit von <60 Sekunden zerfallen. Über die Abluft 9013 kann dann der plasmaaktivierte Wasserdampf beziehungsweise die daraus entstandene Abluft aus dem Beatmungsgerät 10 in die Umgebungsluft ausgeleitet werden.

In manchen Ausführungsformen ist das System 1, beispielsweise über die Erkennungseinheit 108 und/oder die Sensoreinheit 105 zusammen mit der Auswerteeinheit 106, dazu eingerichtet vor der Sterilisation zu testen, ob die Sterilisationsvorrichtung 20 mit der Patientengasführung 901 verbunden ist, beispielsweise über einen kurzen Strom an Arbeitsgas, welcher entsprechend in der Patientengasführung 901 detektiert wird. Schlägt dieser Test fehl kann beispielsweise vorgesehen sein, einen Alarm bzw. Hinweis auszugeben und/oder die Sterilisationsvorrichtung 20 zu sperren. Die Sperre kann dann durch einen erfolgreichen Test aufgehoben werden.

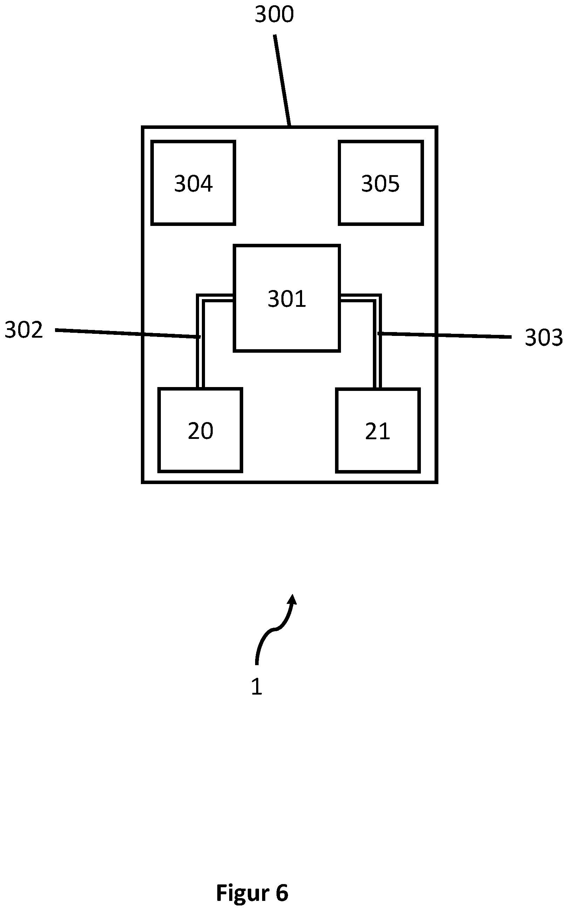

In Figur 6 ist eine beispielhafte Ausführungsform des Systems 1 zu sehen, wobei das medizintechnische Gerät ein Inkubator 300 ist. Der Inkubator kann beispielsweise im Wesentlichen einem aus dem Stand der Technik bekannten Inkubator entsprechen. Beispielhaft ist die Sterilisationsvorrichtung 20 und eine Abgasbehandlung 21 innerhalb des Inkubators 300 vorgesehen, eine externe Anordnung kann aber auch möglich sein. Zudem verfügt der Inkubator 300 über einen Inkubationsraum 301, beispielsweise zur Aufnahme eines Frühgeborenen. Das Klima innerhalb des Inkubationsraumes 301 wird beispielsweise über die Klimaeinheit 305 eingestellt. Die Klimaeinheit 305 kann beispielsweise dazu eingerichtet sein, Luftfeuchtigkeit und/oder Temperatur innerhalb des Inkubationsraumes 301 einzustellen. Es kann auch vorgesehen sein, dass über die Klimaeinheit 305 die Gaszusammensetzung innerhalb des Inkubationsraumes 301 eingestellt werden kann, beispielsweise in Form einer erhöhten Sauerstoffkonzentration.

Über die Zuleitung 302 kann plasmaaktivierter Wasserdampf von der Sterilisationsvorrichtung 20 in den zu sterilisierenden Inkubationsraum 301 geleitet werden. Beispielsweise wird über eine Umwälzeinheit 304 dafür gesorgt, dass der plasmaaktivierte Wasserdampf im gesamten Inkubationsraum 301 verteilt wird und insbesondere die Oberflächen erreicht.

Über eine Ableitung 303 wird der plasmaaktivierte Wasserdampf aus dem Inkubationsraum 301 abgeleitet. Beispielsweise kann vorgesehen sein, dass die Abgasbehandlung 21 eine Vorrichtung zum Absaugen des plasmaaktivierten Wasserdampfs aus dem Inkubationsraum 301 umfasst. Die Abgasbehandlung 21 sorgt weiterhin dafür, dass zumindest die langlebigen Spezies des plasmaaktivierten Wasserdampfs neutralisiert und/oder umgewandelt werden.

In manchen Ausführungsformen umfasst der Inkubator 300 Sicherheitsmaßnahmen, etwa Bewegungssensoren, über welche sichergestellt ist, dass sich zu Beginn der Sterilisation kein Lebewesen, insbesondere kein Frühgeborenes, im Inkubationsraum 301 befindet. Es kann zudem vorgesehen sein, dass zumindest festgestellt wird, dass der Inkubationsraum 301 geschlossen ist, sodass ein ungewolltes Entweichen von plasmaaktiviertem Wasserdampf verhindert wird. Wird festgestellt, dass der Inkubationsraum 301 nicht geschlossen ist und/oder ein Lebewesen detektiert, so kann vorgesehen sein, dass ein Alarm ausgegeben wird du/oder der Beginn der Sterilisation verhindert wird. Es kann zudem vorgesehen sein, dass der Inkubator 300 über Sensoren verfügt, welche das Gas innerhalb des Inkubationsraumes 301 analysieren und darüber festgestellt werden kann, ob der Inkubationsraum 301 frei von gesundheitsschädlichen Rückständen, insbesondere des plasmaaktivierten Wasserdampfes, ist. Werden gesundheitsschädliche Rückstände festgestellt, kann beispielsweise vorgesehen sein, dass, zumindest vor Inbetriebnahme, ein Alarm ausgegeben wird und/oder eine Verwendung des Inkubators 300 gesperrt wird.

### Bezugszeichenliste

- 1: System
- 10: Beatmungsgerät
- 20: Sterilisationsvorrichtung
- 21: Abgasbehandlung
- 101: Gehäuse
- 102: Atemgasquelle
- 103: Fördereinheit
- 104: Steuereinheit
- 105: Sensoreinheit
- 106: Auswerteeinheit
- 107: Benutzerschnittstelle
- 108: Erkennungseinheit
- 109: CO2-Absorber
- 110: Absaugeinheit
- 111: Abgasbehandlung
- 300: Inkubator
- 301: Inkubationsraum
- 302: Zuleitung
- 303: Ableitung
- 304: Umwälzeinheit
- 305: Klimaeinheit
- 901: Patientengasführung
- 9001: Ventil
- 9002: Ventil
- 9003: Ventil
- 9004: Ventil
- 9005: Ventil
- 9006: Ventil
- 9007: Ventil
- 9008: Ventil
- 9009: Ventil
- 9011: Inspirationsanschluss
- 9012: Exspirationsanschluss
- 9013: Abluft
- 9014: Patientenanschluss
- 9015: Sterilisationsanschluss
- 9016: Absauganschluss
- 9017: Exspirationsweg
- 9018: Inspirationsweg

## Patentansprüche

1. System (1) zur Sterilisation eines medizintechnischen Gerätes, umfassend zumindest ein medizintechnisches Gerät und zumindest eine Sterilisationsvorrichtung (20), wobei die Sterilisationsvorrichtung (20) zumindest einen Plasmagenerator und zumindest eine Wasserquelle und/oder Wasserdampfquelle zur Bereitstellung von Wasser und/oder Wasserdampf umfasst, wobei die Sterilisationsvorrichtung (20) dazu ausgebildet ist, plasmaaktivierten Wasserdampf zu generieren und, dass das System (1) dazu eingerichtet ist, den plasmaaktivierten Wasserdampf zumindest teilweise durch das medizintechnische Gerät zu leiten,
**dadurch gekennzeichnet, dass** das medizintechnische Gerät ein Beatmungsgerät (10) ist, wobei das Beatmungsgerät (10) zumindest eine Patientengasführung (901) umfasst und das System (1) dazu eingerichtet ist, den plasmaaktivierten Wasserdampf zumindest teilweise durch die Patientengasführung (901) zu leiten.

2. System (1) nach Anspruch 1, **dadurch gekennzeichnet, dass** das System (1) eine Abgasbehandlung (21, 111) umfasst, wobei die Abgasbehandlung (21, 111) zumindest einen Filter und/oder einen Plasmagenerator umfasst und dazu eingerichtet und ausgebildet ist, den plasmaaktivierten Wasserdampf aufzubereiten.

3. System (1) nach Anspruch 2, **dadurch gekennzeichnet, dass** die Abgasbehandlung (21, 111) dazu eingerichtet und ausgebildet ist, aktive Spezies aus dem plasmaaktivierten Wasserdampf herauszufiltern.

4. System (1) nach zumindest einem der vorhergehenden Ansprüche 2 oder 3, **dadurch gekennzeichnet, dass** die Abgasbehandlung (21, 111) dazu eingerichtet und ausgebildet ist, langlebige aktive Spezies in kurzlebige Spezies umzuwandeln.

5. System (1) nach zumindest einem der vorhergehenden Ansprüche, **dadurch gekennzeichnet, dass** die Sterilisationsvorrichtung (20) in das Beatmungsgerät (10) integriert ist.

6. System (1) nach zumindest einem der vorhergehenden Ansprüche, **dadurch gekennzeichnet, dass** das Beatmungsgerät (10) eine Atemgasquelle (102) umfasst, wobei die Atemgasquelle (102) mit der Sterilisationsvorrichtung (20) verbunden ist und dazu eingerichtet ist, die Sterilisationsvorrichtung (20) mit Arbeitsgas zu versorgen.

7. System (1) nach zumindest einem der vorhergehenden Ansprüche, **dadurch gekennzeichnet, dass** die Sterilisationsvorrichtung (20) zur Sterilisation mit einem Exspirationsanschluss (9012) und/oder einem Inspirationsanschluss (9011) der Patientengasführung (901) verbindbar ist und das System (1) so eingerichtet und ausgebildet ist, dass während der Sterilisation plasmaaktivierter Wasserdampf von der Sterilisationsvorrichtung (20) durch den Exspirationsanschluss (9012) und/oder den Inspirationsanschluss (9011) in die Patientengasführung (901) geleitet wird.

8. System (1) nach Anspruch 7, **dadurch gekennzeichnet, dass** die Sterilisationsvorrichtung (20) während der Sterilisation so mit der Patientengasführung (901) verbunden ist, dass der plasmaaktivierte Wasserdampf durch den Exspirationsanschluss (9012) in die Patientengasführung (901) eingeleitet wird und durch den Inspirationsanschluss (9011) aus der Patientengasführung (901) ausgeleitet wird, wobei die Patientengasführung (901) so ausgebildet und eingerichtet ist, dass der plasmaaktivierte Wasserdampf bei der Strömung von Exspirationsanschluss (9012) zu Inspirationsanschluss (9011) die gesamte Patientengasführung (901) durchströmt.

9. System (1) nach zumindest einem der vorhergehenden Ansprüche, **dadurch gekennzeichnet, dass** das Beatmungsgerät (10) eine Absaugeinheit (110) umfasst, welche dazu eingerichtet und ausgebildet ist, den plasmaaktivierten Wasserdampf durch die Patientengasführung (901) zu saugen.

10. System (1) nach zumindest einem der vorhergehenden Ansprüche 7 oder 8, **dadurch gekennzeichnet, dass** die Abgasbehandlung (21, 111) in das Beatmungsgerät (10) integriert ist, wobei die Abgasbehandlung (21, 111) dazu eingerichtet und ausgebildet ist, den plasmaaktivierten Wasserdampf, welcher durch den Inspirationsanschluss (9011) und/oder den Exspirationsanschluss (9012) aus der Patientengasführung (901) ausgeleitet wird, aufzubereiten.

11. System (1) nach zumindest einem der vorhergehenden Ansprüche, **dadurch gekennzeichnet, dass** die Sterilisationsvorrichtung (20) und die Abgasbehandlung (21, 111) innerhalb des Beatmungsgerätes (10) mit der Patientengasführung (901) so verbunden ist, dass bei der Sterilisation plasmaaktivierter Wasserdampf durch die gesamte Patientengasführung (901) strömt.

12. System (1) nach zumindest einem der vorhergehenden Ansprüche, **dadurch gekennzeichnet, dass** in dem System (1) eine Benutzerschnittstelle (107) angeordnet ist und über die Benutzerschnittstelle (107) eine Dauer und/oder ein Modus der Sterilisation einstellbar ist.

13. System (1) nach zumindest einem der vorhergehenden Ansprüche, **dadurch gekennzeichnet, dass** das Beatmungsgerät (10) dazu eingerichtet ist, die Voraussetzungen zum Start der Sterilisation zu prüfen und nur dann die Sterilisation freigibt, wenn alle Voraussetzungen erfüllt sind.

14. System (1) nach zumindest einem der vorhergehenden Ansprüche, **dadurch gekennzeichnet, dass** die Sterilisationsvorrichtung (20) dazu eingerichtet ist in einem ersten Sterilisationsmodus einen dauerhaften Strom an plasmaaktiviertem Wasserdampf bereitzustellen und/oder durch die Patientengasführung (901) zu fördern.

15. System (1) nach zumindest einem der vorhergehenden Ansprüche, **dadurch gekennzeichnet, dass** die Sterilisationsvorrichtung (20) dazu eingerichtet ist, in einem zweiten Sterilisationsmodus einen gepulsten Strom an plasmaaktiviertem Wasserdampf bereitzustellen und/oder durch die Patientengasführung (901) zu fördern.

16. System (1) nach zumindest einem der vorhergehenden Ansprüche, **dadurch gekennzeichnet, dass** das medizintechnische Gerät ein Inkubator (300) ist, wobei der Inkubator (300) zumindest einen Inkubationsraum (301) aufweist und der Inkubationsraum (301) zumindest eine Zuleitung (302) und eine Ableitung (303) verfügt und über die Zuleitung (302) plasmaaktivierter Wasserdampf in den Inkubationsraum (301) geleitet wird und über die Ableitung (303) abgleitet wird.

17. Verfahren zur Sterilisation eines medizintechnischen Gerätes, wobei über eine Sterilisationsvorrichtung (20) ein plasmaaktivierter Wasserdampf generiert wird und der plasmaaktivierte Wasserdampf zumindest teilweise durch das medizintechnische Gerät geleitet wird,
**dadurch gekennzeichnet, dass** das medizintechnische Gerät ein Beatmungsgerät (10) ist und der plasmaaktivierte Wasserdampf zumindest teilweise durch eine Patientengasführung (901) des Beatmungsgerätes (10) geleitet wird.

18. Verfahren nach Anspruch 17, **dadurch gekennzeichnet, dass** der plasmaaktivierte Wasserdampf durch die gesamte Patientengasführung (901) geleitet wird, wobei der plasmaaktivierte Wasserdampf nach Strömung durch die Patientengasführung (901) durch eine Abgasbehandlung (111) geleitet wird.

## Claims

1. A system (1) for sterilizing a medical device, comprising at least one medical device and at least one sterilization device (20), wherein the sterilization device (20) comprises at least one plasma generator and at least one water source and/or water vapor source for providing water and/or water vapor, wherein the sterilization device (20) is designed to generate plasma-activated water vapor and that the system (1) is configured to conduct the plasma-activated water vapor at least partially through the medical device,
**characterized in that** the medical device is a ventilator (10), wherein the ventilator (10) comprises at least one patient gas guide (901) and the system (1) is configured to conduct the plasma-activated water vapor at least partially through the patient gas guide (901).

2. The system (1) according to claim 1, **characterized in that** the system (1) comprises a waste gas treatment (21, 111), wherein the waste gas treatment (21, 111) comprises at least one filter and/or a plasma generator and is configured and designed to treat the plasma-activated water vapor.

3. The system (1) according to claim 2, **characterized in that** the waste gas treatment (21, 111) is configured and designed to filter reactive species out of the plasma-activated water vapor.

4. The system (1) according to at least one of the preceding claims 2 or 3, **characterized in that** the waste gas treatment (21, 111) is configured and designed to convert long-lived reactive species into short-lived reactive species.

5. The system (1) according to at least one of the preceding claims, **characterized in that** the sterilization device (20) is integrated into the ventilator (10).

6. The system (1) according to at least one of the preceding claims, **characterized in that** the ventilator (10) comprises a respiratory gas source (102), wherein the respiratory gas source (102) is connected to the sterilization device (20) and is configured to supply the sterilization device (20) with working gas.

7. The system (1) according to at least one of the preceding claims, **characterized in that** the sterilization device (20) can be connected for sterilization to an expiration port (9012) and/or an inspiration port (9011) of the patient gas guide (901) and the system (1) is configured and designed such that, during sterilization, plasma-activated water vapor is conducted from the sterilization device (20) into the patient gas guide (901) through the expiration port (9012) and/or the inspiration port (9011).

8. The system (1) according to claim 7, **characterized in that,** during sterilization, the sterilization device (20) is connected to the patient gas guide (901) such that the plasma-activated water vapor is introduced into the patient gas guide (901) through the expiration port (9012) and is discharged out of the patient gas guide (901) through the inspiration port (9011), wherein the patient gas guide (901) is designed and configured such that, when the plasma-activated water vapor flows from the expiration port (9012) to the inspiration port (9011), it flows through the entire patient gas guide (901).

9. The system (1) according to at least one of the preceding claims, **characterized in that** the ventilator (10) comprises a suction unit (110), which is configured and designed to suck the plasma-activated water vapor through the patient gas guide (901).

10. The system (1) according to at least one of the preceding claims 7 or 8, **characterized in that** the waste gas treatment (21, 111) is integrated into the ventilator (10), wherein the waste gas treatment (21, 111) is configured and designed to treat the plasma-activated water vapor which is discharged from the patient gas guide (901) through the inspiration port (9011) and/or the expiration port (9012).

11. The system (1) according to at least one of the preceding claims, **characterized in that** the sterilization device (20) and the waste gas treatment (21, 111) within the ventilator (10) are connected to the patient gas guide (901) such that, during sterilization, plasma-activated water vapor flows through the entire patient gas guide (901).

12. The system (1) according to at least one of the preceding claims, **characterized in that** a user interface (107) is arranged in the system (1) and a duration and/or a mode of sterilization can be set using the user interface (107).

13. The system (1) according to at least one of the preceding claims, **characterized in that** the ventilator (10) is configured to check the requirements at the start of sterilization and enables the sterilization only when all the requirements have been met.

14. The system (1) according to at least one of the preceding claims, **characterized in that** the sterilization device (20) is configured in a first sterilization mode to provide a continuous flow of plasma-activated water vapor and/or to convey said flow through the patient gas guide (901).

15. The system (1) according to at least one of the preceding claims, **characterized in that** the sterilization device (20) is configured in a second sterilization mode to provide a pulsed flow of plasma-activated water vapor and/or to convey said flow through the patient gas guide (901).

16. The system (1) according to at least one of the preceding claims, **characterized in that** the medical device is an incubator (300), wherein the incubator (300) has at least one incubation space (301) and the incubation space (301) has at least one supply line (302) and one discharge line (303) and plasma-activated water vapor is conducted into the incubation space (301) via the supply line (302) and discharged via the discharge line (303).

17. A method for sterilizing a medical device, wherein a plasma-activated water vapor is generated by a sterilization device (20) and the plasma-activated water vapor is conducted at least partially through the medical device,
**characterized in that** the medical device is a ventilator (10) and the plasma-activated water vapor is conducted at least partially through a patient gas guide (901) of the ventilator (10).

18. The method according to claim 17, **characterized in that** the plasma-activated water vapor is conducted through the entire patient gas guide (901), wherein the plasma-activated water vapor is conducted through a waste gas treatment (111) after flowing through the patient gas guide (901).

## Revendications

1. Système (1) pour la stérilisation d'un appareil médical, comportant au moins un appareil médical et au moins un dispositif de stérilisation (20), dans lequel le dispositif de stérilisation (20) comporte au moins un générateur de plasma et au moins une source d'eau et/ou une source de vapeur d'eau pour l'approvisionnement en eau et/ou en vapeur d'eau, dans lequel le dispositif de stérilisation (20) est conçu pour générer de la vapeur d'eau activée par plasma, et le système (1) est configuré pour guider la vapeur d'eau activée par plasma au moins partiellement à travers l'appareil médical,
**caractérisé en ce que** l'appareil médical est un appareil de ventilation (10), dans lequel l'appareil de ventilation (10) comporte au moins une conduite de gaz pour patient (901) et le système (1) est configuré pour guider la vapeur d'eau activée par plasma au moins partiellement à travers la conduite de gaz pour patient (901).

2. Système (1) selon la revendication 1, **caractérisé en ce que** le système (1) comporte un traitement de gaz d'échappement (21, 111), dans lequel le traitement de gaz d'échappement (21, 111) comporte au moins un filtre et/ou un générateur de plasma et est configuré et conçu pour la préparation de la vapeur d'eau activée par plasma.

3. Système (1) selon la revendication 2, **caractérisé en ce que** le traitement de gaz d'échappement (21, 111) est configuré et conçu pour filtrer des espèces actives hors de la vapeur d'eau activée par plasma.

4. Système (1) selon l'une au moins des revendications précédentes 2 ou 3, **caractérisé en ce que** le traitement de gaz d'échappement (21, 111) est configuré et conçu pour transformer des espèces actives à longue durée de vie en espèces actives à courte durée de vie.

5. Système (1) selon l'une au moins des revendications précédentes, **caractérisé en ce que** le dispositif de stérilisation (20) est intégré dans l'appareil de ventilation (10).

6. Système (1) selon l'une au moins des revendications précédentes, **caractérisé en ce que** l'appareil de ventilation (10) comporte une source de gaz respirable (102), dans lequel la source de gaz respirable (102) est reliée au dispositif de stérilisation (20) et configurée pour alimenter le dispositif de stérilisation (20) en gaz de travail.

7. Système (1) selon l'une au moins des revendications précédentes, **caractérisé en ce que** le dispositif de stérilisation (20) peut être relié à un raccord d'expiration (9012) et/ou à un raccord d'inspiration (9011) de la conduite de gaz pour patient (901) pour la stérilisation et le système (1) est configuré et conçu de telle façon que pendant la stérilisation, de la vapeur d'eau activée par plasma est guidée à partir du dispositif de stérilisation (20) vers la conduite de gaz pour patient (901) à travers le raccord d'expiration (9012) et/ou le raccord d'inspiration (9011).

8. Système (1) selon la revendication 7, **caractérisé en ce que** le dispositif de stérilisation (20) est relié à la conduite de gaz pour patient (901) pendant la stérilisation, **en ce que** la vapeur d'eau activée par plasma est introduite dans la conduite de gaz pour patient (901) à travers le raccord d'expiration (9012) et évacuée de la conduite de gaz pour patient (901) à travers le raccord d'inspiration (9011), dans lequel la conduite de gaz pour patient (901) est conçue et configurée de telle façon que de la vapeur d'eau activée par plasma s'écoule à travers l'ensemble de la conduite de gaz pour patient (901) lors de l'écoulement du raccord d'expiration (9012) au raccord d'inspiration (9011).

9. Système (1) selon l'une au moins des revendications précédentes, **caractérisé en ce que** l'appareil de ventilation (10) comporte une unité d'aspiration (110), laquelle est configurée et conçue pour aspirer la vapeur d'eau activée par plasma à travers la conduite de gaz pour patient (901).

10. Système (1) selon l'une au moins des revendications précédentes 7 ou 8, **caractérisé en ce que** le traitement de gaz d'échappement (21, 111) est intégré dans l'appareil de ventilation (10), dans lequel le traitement de gaz d'échappement (21, 111) est configuré et conçu pour préparer la vapeur d'eau activée par plasma évacuée hors de la conduite de gaz pour patient (901) à travers le raccord d'inspiration (9011) et/ou le raccord d'expiration (9012).

11. Système (1) selon l'une au moins des revendications précédentes, **caractérisé en ce que** le dispositif de stérilisation (20) et le traitement de gaz d'échappement (21, 111) sont reliés de telle façon à la conduite de gaz pour patient (901) à l'intérieur de l'appareil de ventilation (10), que de la vapeur d'eau activée par plasma s'écoule à travers l'ensemble de la conduite de gaz pour patient (901) pendant la stérilisation.

12. Système (1) selon l'une au moins des revendications précédentes, **caractérisé en ce qu'**une interface d'utilisateur (107) est disposée dans le système (1) et une durée et/ou un mode de stérilisation peuvent être réglés par le biais de l'interface d'utilisateur (107).

13. Système (1) selon l'une au moins des revendications précédentes, **caractérisé en ce que** l'appareil de ventilation (10) est configuré pour vérifier les conditions de démarrage de la stérilisation et n'autorise la stérilisation que lorsque toutes les conditions sont remplies.

14. Système (1) selon l'une au moins des revendications précédentes, **caractérisé en ce que** dans un premier mode de stérilisation, le dispositif de stérilisation (20) est configuré pour fournir et/ou transporter, à travers la conduite de gaz pour patient (901), un flux durable de vapeur d'eau activée par plasma.

15. Système (1) selon l'une au moins des revendications précédentes, **caractérisé en ce que** dans un deuxième mode de stérilisation, le dispositif de stérilisation (20) est configuré pour fournir et/ou transporter, à travers la conduite de gaz pour patient (901), un flux pulsé de vapeur d'eau activée par plasma.

16. Système (1) selon l'une au moins des revendications précédentes, **caractérisé en ce que** l'appareil médical est un incubateur (300), dans lequel l'incubateur (300) présente au moins un espace d'incubation (301) et l'espace d'incubation (301) est doté d'au moins une conduite d'amenée (302) et une conduite d'évacuation (303) et de la vapeur d'eau activée par plasma est guidée vers l'espace d'incubation (301) par le biais de la conduite d'amenée (302) et évacuée par le biais de la conduite d'évacuation (303).

17. Procédé de stérilisation d'un appareil médical, dans lequel une vapeur d'eau activée par plasma est générée par un dispositif de stérilisation (20) et la vapeur d'eau activée par plasma est guidée au moins partiellement à travers l'appareil médical,
**caractérisé en ce que** l'appareil médical est un appareil de ventilation (10) et la vapeur d'eau activée par plasma est guidée au moins partiellement à travers une conduite de gaz pour patient (901) de l'appareil de ventilation (10).

18. Procédé selon la revendication 17, **caractérisé en ce que** la vapeur d'eau activée par plasma est guidée à travers l'ensemble de la conduite de gaz pour patient (901), dans lequel la vapeur d'eau activée par plasma est guidée à travers un traitement de gaz d'échappement (111) après écoulement à travers la conduite de gaz pour patient (901).
